# EUROPEAN PATENT SPECIFICATION

(11) **EP 2 950 095 B1**
(45) Date of publication and mention of the grant of the patent: **29.08.2018**
(21) Application number: 14170362.9
(22) Date of filing: 28.05.2014
(51) Int. Cl.: G01N 33/50

(54) **Cell-based assay and screening methods for modulators of p75NTR signaling**
Zellenbasierte Test- und Screeningverfahren auf Modulatoren von p75NTR-Signalisierung
Analyse cellulaire et procédés de criblage de modulateurs de signalisation de p75NTR

(43) Date of publication of application: 02.12.2015
(73) Proprietor: Technische Universität Dresden, 01069 Dresden (DE)
(72) Inventor: Brenner, Sebastian, 01309 Dresden (DE); Ryser, Martin, 1050 Ixelles (BE); Richter, Cornelia, 01139 Dresden (DE); Thieme, Sebastian, 01127 Dresden (DE)
(74) Representative: Maikowski & Ninnemann Patentanwälte Partnerschaft mbB

(56) References cited:
- WO-A1-97/37228
- WO-A1-2012/101664
- US-A1- 2004 171 086
- US-A1- 2008 064 036
- SANTANA-DE ANDA KARINA ET AL: "Plasmacytoid dendritic cells: Key players in viral infections and autoimmune diseases", SEMINARS IN ARTHRITIS AND RHEUMATISM, vol. 43, no. 1, 22 February 2013 (2013-02-22), pages 131-136, XP028690819, ISSN: 0049-0172, DOI: 10.1016/J.SEMARTHRIT.2012.12.026
- NING YU ET AL: "Cultured human melanocytes express functional Toll-like receptors 2-4, 7 and 9", JOURNAL OF DERMATOLOGICAL SCIENCE, vol. 56, no. 2, 1 November 2009 (2009-11-01), pages 113-120, XP055155572, ISSN: 0923-1811, DOI: 10.1016/j.jdermsci.2009.08.003
- Y. JIANG ET AL: "Nerve Growth Factor Promotes TLR4 Signaling-Induced Maturation of Human Dendritic Cells In Vitro through Inducible p75", THE JOURNAL OF IMMUNOLOGY, vol. 179, no. 9, 18 October 2007 (2007-10-18), pages 6297-6304, XP055155667, ISSN: 0022-1767, DOI: 10.4049/jimmunol.179.9.6297

## Description

### Field of the invention

The present invention relates generally to immune reactions and testing substances useful for same. Specifically, the present invention relates to the modulation of the nerve growth factor receptor p75NTR, which is expressed by human and murine plasmacytoid dendritic cells. The invention provides assays to screen for a range of agonists and antagonists useful in modulating cytokine function, antigen presentation, T-cell activation and proliferation. The present invention further provides, therefore, screening assays for agonists and antagonists of p75NTR-modulated immune responses. Such agonists and antagonists are useful in the manufacture of drugs for controlling cytokine function, antigen presentation, T-cell activation and proliferation, which is important for the treatment of a range of conditions including cancer, inflammatory reactions, immunological disorders, growth disorders and any other conditions involving p75NTR signal transduction. The invention further provides a combination comprising at least one modulator of p75^{NTR} signalling in PDCs selected from a p75^{NTR} antagonist or p75^{NTR} agonist and at least one agonist of TLR9.

### Background of the invention

The immune system functions to protect individuals from infective agents, e.g., bacteria, multicellular organisms, and viruses, as well as from cancers. This system includes several types of lymphoid and myeloid cells such as T-cells, B-cells, monocytes, macrophages, dendritic cells (DCs), eosinophils and neutrophils. These lymphoid and myeloid cells often produce signaling proteins known as cytokines. The immune response includes inflammation, i.e., the accumulation of immune cells systemically or in a particular location of the body and can lead to autoimmune disease or graft versus host disease. In response to an infective agent or foreign substance, immune cells secrete cytokines which, in turn, modulate immune cell proliferation, development, differentiation, or migration. Cytokines have been implicated in the pathology of a number of disorders and conditions.

In more detail, the human immune system has developed to give us protection against microbes by coordination of innate (non-specific) and adaptive/acquired immune mechanisms (combination of cell mediated and humoral immune responses). The innate immunity cells include epithelial cells, NK (natural killer) cells and PDC (plasmacytoid dendritic cells). These cells function as first line of body defense against any attacking microbes by secreting antimicrobial cytokines e.g. on viral encounter PDC secret type I interferons (IFN), a family of anti-viral cytokines with potent anti-viral activity. Adaptive immunity, on the other hand, includes T helper cells (Th1 and Th2) and cytotoxic T cell based immune responses (cell mediated immunity) and B-cells that differentiate into antigen specific antibody producing B plasma cells (humoral immunity). PDC, in addition to their vital role in innate immunity, have the ability to trigger T cell responses and regulate B cell growth and differentiation into antibody secreting plasma cells. PDC contribute essentially in regulating and bridging antigen induced innate and adaptive immune responses.

PDC express endosomal toll like receptors (TLR) and are in fact the only human cells that express toll like receptors 7 (TLR7) and 9 (TLR9) that are able to bind single stranded viral RNA and bacterial or viral DNA, respectively. Upon activation of TLR7 and TLR9, a signaling cascade is activated involving MyD88, TRAF6, IRF3 and IRF7, which ultimately leads to the production of very high levels of interferon alpha. Interferon alpha induces Th1 immune reactions and has multiple functions in the human body in viral defense, in the elimination of tumor cells, but also in the induction of autoimmunity. For a long time interferon production upon toll like receptor activation associated with the induction of a Th1 immune reaction seemed to be the only function that could be attributed to PDCs.

TRAF3 and TRAF6 are human protein members of the TNF receptor associated factor (TRAF) protein family. TRAF proteins are associated with, and mediate signal transduction from members of the TNF receptor superfamily. These proteins mediate the signaling not only from the members of the TNF receptor superfamily, but also from the members of the Toll/IL-1 family.

Loss of Myeloid Differentiation primary response gene 88 (MyD88) expression is associated with decreased resistance to bacterial infections. Moreover, mutated forms of MyD88 have been identified in various human lymphomas (Hawn et al., J Infect Dis. (2006) 193 (12): 1693-1702).

Interferon regulatory factor (IRF) 3 and 7 are members of the interferon regulatory factor family of transcription factors. IRF3 and 7 have been shown to play a role in the transcriptional activation of virus-inducible cellular genes, including pro-inflammatory and type I interferon genes. In particular, IRF7 regulates many interferon-alpha genes. Constitutive expression of IRF7 is largely restricted to lymphoid tissue, particularly PDCs. Expression of IRF7 is, however, inducible in many tissues.

Neurotrophins are the family of proteins which are considered to have an essential role in the development of the vertebrate nervous system. Nerve growth factor (NGF) is the best characterized member of the neurotrophin family and was the first to be isolated. Other members of the ever growing family of neurotrophins include: Brain derived nerve factor (BDNF), Neurotrophin-3 (NT-3) and Neurotrophin-4 and 5 (NT-4 and NT-5). Neurotrophins mediate their effects by binding to two different receptors classes with different affinities: i) high affinity nerve growth factor receptor which includes: the Trk A, Trk B and Trk C (tropomyosin-receptor kinase A, B and C), and ii) low affinity nerve growth factor receptor (LNGFR), member of the tumor necrosis factor receptor superfamily, which is also known as p75NTR or CD271 (Lykissas et al., Curr Neurovasc Res. 2007 May;4(2):143-51).

In recent years it has been demonstrated that PDCs also play a pivotal role in the regulation of immune responses to exogenous antigens and self-antigens. It could be demonstrated that depletion of PDCs in mice aggravates allergic Asthma, which is a Th2 immune response, but also worsens the autoimmune reaction in experimental autoimmune encephalomyelitis, a mouse model for multiple sclerosis, which is based on a Th1 immune response. From those results it could be deducted that PDCs have a major regulatory function to induce tolerance, but might also be involved in the escape of tumor cells from host immunity.

The induction of immune reaction and the inhibition of tolerance are major determinants for the success of vaccination strategies. Classical vaccines rely on the induction of Th2 immune reactions to induce humoral immunity against the vaccine antigens. As attenuated vaccines do not induce a strong immune reaction, adjuvants are used to potentiate the immune response. The most common Th2 inducing adjuvants are aluminum salts. In order to kill intracellular organisms or to eliminate tumor cells, Th1 cytotoxic immune responses need to be induced, for which therefore different adjuvants are to be used. Most of the Th1 inducing adjuvants act via activation of toll like receptors. An overview on current adjuvants or new adjuvants that are being evaluated in clinical trials are shown in table 1 below:

**Table 1.**

| **Adjuvant name** | **Class** | **Mechanism or receptor** | **Type of immune response** | **Clinical phase or licensed product name** |
|---|---|---|---|---|
| dsRNA analogues (for example, poly(I:C)) | IM | TLR3 | Ab, Th1, CD8+ T-cells | Phase 1 |
| Lipid A analogues (for example, MPL, RC529, GLA, E6020) | IM | TLR4 | Ab, Th1 | Cervarix, Supervax, Pollinex Quattro, Melacine |
| Flagellin | IM | TLR5 | Ab, Thl,Th2 | Phase 1 |
| Imidazoquinolines (for example, Imiquimod, R848) | IM | TLR7 and TLR8 | Ab, Th1 | Aldara |
| CpG ODN | IM | TLR9 | Ab, Th1, CD8+ T-cells | Phase 3 |
| Saponins (for example, QS21) | IM | Unknown | Ab, Thl,Th2, CD8+ T-cells | Phase 3 |
| C-type lectin ligands (for example, TDB ) | IM | Mincle, Nalp3 | Ab, Th1, Th17 | Phase 1 |
| CDld ligands (for example, α-galactosylceramide) | IM | CD1d | Ab, Th1, Th2, CD8+ NKT cells | Phase 1 |
| Aluminum salts (for example, aluminum oxyhydroxide, aluminum phosphate) | PF | Nalp3, ITAM, Ag delivery | Ab, Th2 | Numerous licensed products |
| Emulsions (for example, MF59, AS03, AF03, SE) | PF | Immune cell recruitment, ASC, Ag uptake | Ab, Th1, Th2 | Fluad, Pandemrix |
| Virosomes | PF | Ag delivery | Ab, Th1, Th2 | Epaxal, Inflexal V |
| AS01 (MPL, QS21, liposomes) | c | TLR4 | Ab, Th1, CD8+ T-cells | Phase 3 |
| AS02 (MPL, QS21, emulsion) | C | TLR4 | Ab, Th1 | Phase 3 |
| AS04 (MPL, aluminum salt) | C | TLR4 | Ab, Th1 | Cervarix |
| AS15 (MPL, QS21, CpG, liposomes) | C | TLR4 and TLR9 | Ab, Th1, CD8+ T-cells | Phase 3 |
| GLA-SE (GLA, emulsion) | C | TLR4 | Ab, Th1 | Phase 1 |
| IC31 (CpG, cationic peptide) | C | TLR9 | Ab, Th1, Th2, CD8+ T-cells | Phase 1 |
| CAF01 (TDB, cationic liposomes) | C | Mincle, Ag delivery | Ab, Th1, CD8+ T-cells | Phase 1 |
| ISCOMs (saponin, phospholipid) | C | Unknown | Ab, Th1, Th2, CD8+ T-cells | Phase 2 |
| Ab, antibody; Ag, antigen; ASC, apoptosis-associated speck-like protein containing caspase recruitment domain; C, combination of immunomodulatory molecule and particulate formulation; dsRNA, double-stranded RNA; IM, immunomodulatory molecule; ITAM, immunoreceptor tyrosine-based activation motif; PF, particulate formulation; TDB, trehalose dibehenate. Some particulate formulations (such as aluminum salts and emulsions) also generate immunomodulatory activity. | | | | |

### Summary of the invention

The invention is based on the unexpected finding that plasmacytoid dendritic cells express the nerve growth factor receptor p75NTR. It could further be established that p75NTR is an important regulator of PDC driven immune responses. P75NTR activation on PDCs strongly induces Th2 immune responses and inhibits Th1 responses.

The invention provides a combination comprising at least one modulator of p75^{NTR} signalling in PDCs selected from a p75^{NTR} antagonist or p75^{NTR} agonist and at least one agonist of TLR9, wherein the agonist or antagonist modulates immune response and/or cell proliferation.

In another aspect, the invention provides said combination for use in treating a subject suffering from a disease or pathological condition that involves p75NTR signaling, such as an inflammatory disorder, immune disorder or cancer, wherein the disease or pathological condition is mediated by monocytes or macrophages, neutrophils, T-cells or B-cells, dendritic cells, or epithelial or endothelial cells. In a further embodiment, the disease is mediated via PDCs.

Another embodiment of the present invention provides a method of screening for a compound that modulates a physiological activity, comprising contacting a candidate compound to an p75NTR knockout mouse, and determining the physiological activity in the contacted p75NTR knockout mouse; determining the physiological activity in an p75NTR knockout mouse not contacted with the candidate compound; and comparing the physiological activities of the contacted p75NTR knockout mouse and the non-contacted p75NTR knockout mouse; as well as the above method wherein the physiological activity comprises an immune activity; inflammation, hyperreactivity, or a proliferative activity.

P75NTR on plasmacytoid dendritic cells seems to function as a master switch in the regulation of immune responses. The modulation of immune responses is the major function of vaccine adjuvants. Therefore agonist and antagonists of p75NTR provide a means for novel adjuvants.

Activation of p75NTR on PDCs strongly induces Th2 immune responses. Therefore agonists can boost immunization responses in Th2 dependent vaccines. The directed immune response is similar to aluminum salts but based without inducing local inflammation. P75NTR agonists might be used to replace current vaccine adjuvants or could be used in combination to further boost a vaccine response.

Accordingly, there is a great need for the identification of novel p75NTR agonists and antagonists. These novel p75NTR agonists and antagonists, in particular the p75NTR agonists can further be used as vaccine adjuvants.

The invention thus further relates to the use of agonists and antagonists of p75NTR signaling as vaccine adjuvant.

The invention further provides vaccine compositions comprising an agonist or antagonist of p75NTR signaling.

In another embodiment the invention relates to the use of a vaccine composition comprising a p75NTR agonist for modulating immune responses comprising but not limited to stimulation of Th2 immune responses, suppression of Th1 immune responses, suppression of Th17 immune responses and suppression of regulatory T-cell induced tolerance.

In yet another embodiment the invention relates to the use of a vaccine composition comprising a p75NTR antagonist for modulating immune responses comprising but not limited to suppression of Th2 immune responses, stimulation of Th1 immune responses, stimulation of Th17 immune responses, suppression of regulatory T-cell induced tolerance and the like.

The invention further provides activators of dendritic cells, preferably of PDCs, and a combination of these activators with modulators, i.e. agonists or antagonists, of p75NTR signaling.

These combinations of activators of dendritic cells with agonists or antagonists of p75NTR signaling can be incorporated into pharmaceutical compositions, preferably in vaccine compositions, for use in immunotherapy.

### Brief description of the drawings

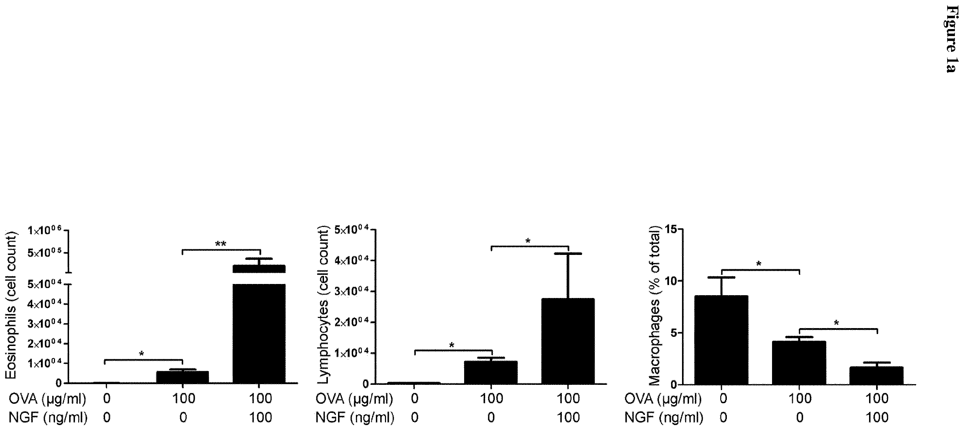

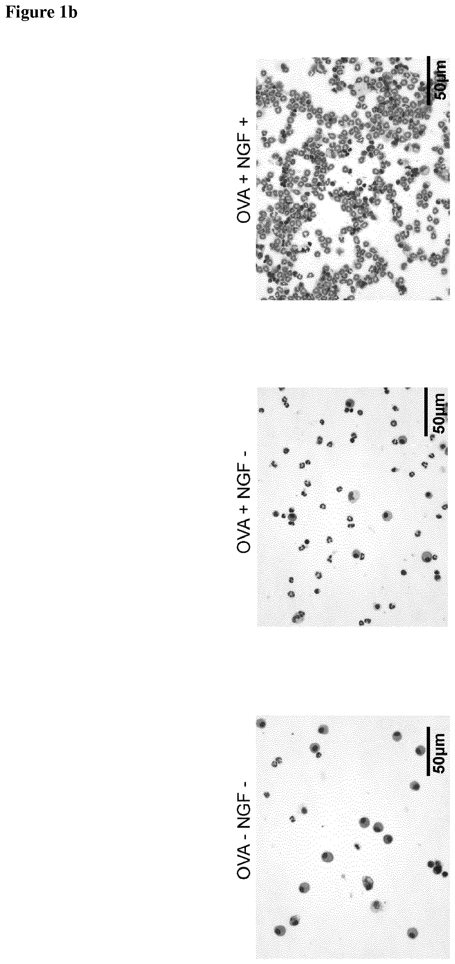

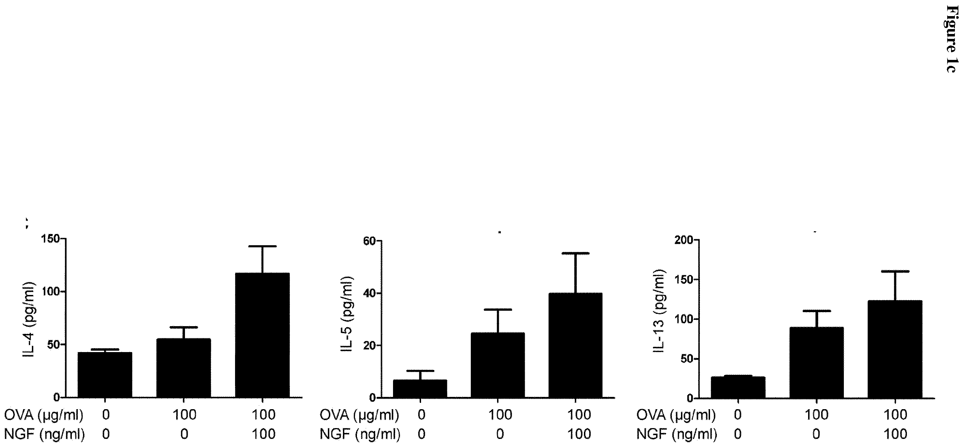

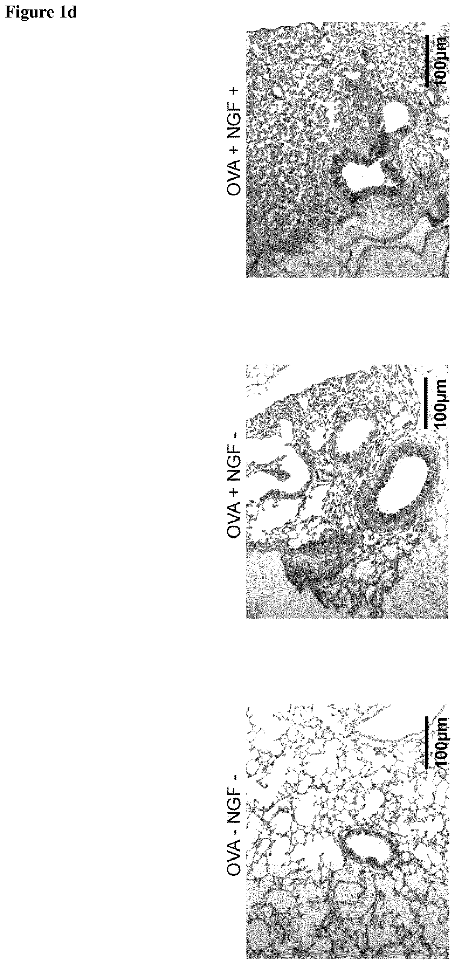

- **Figure 1**: shows the effect of NGF on murine PDCs during allergen-mediated immune response. In the bronchoalveolar lavage fluid (BALF), numbers of eosinophils and lymphocytes were significantly augmented when the OVA up-take by PDCs was carried out in the presence of NGF compared to PDCs incubated with OVA alone, whereas number of macrophages decreased (Fig. 1a, b). OVA-loaded PDCs treated with NGF caused increased production of Th2 cytokines (IL-4, IL-5 and IL-13) in the lung in comparison to PDCs pulsed with OVA in the absence of NGF (Fig. 1c). Histological lung sections from mice that received OVA-loaded PDCs showed increased perivascular inflammation and enhanced mucus production (Fig. 1d). Treatment of PDCs with NGF during OVA-uptake potentiated the inflammatory phenotype in the lung (Fig. 1d).

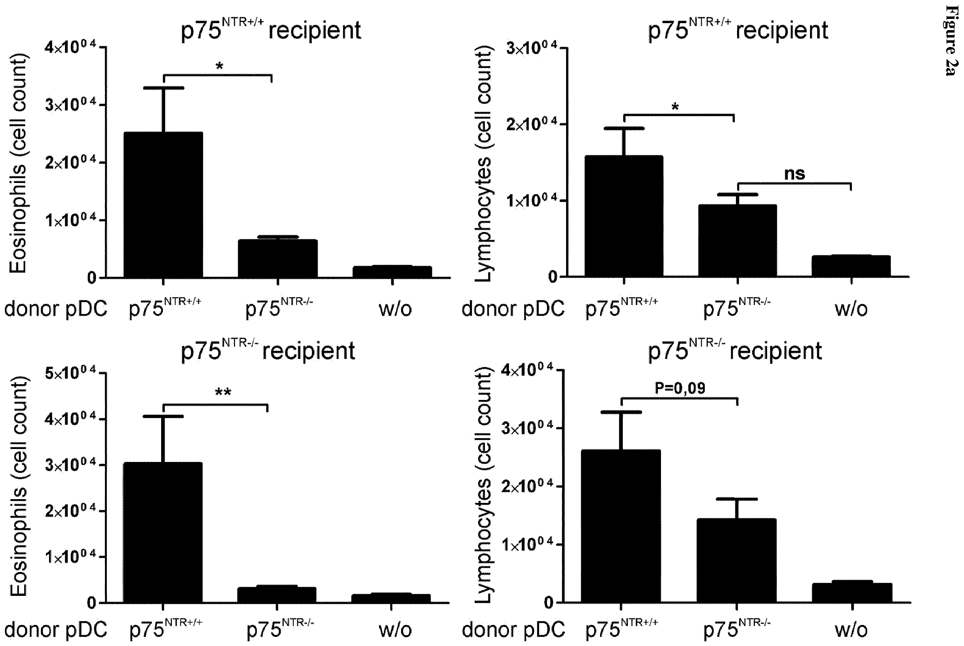

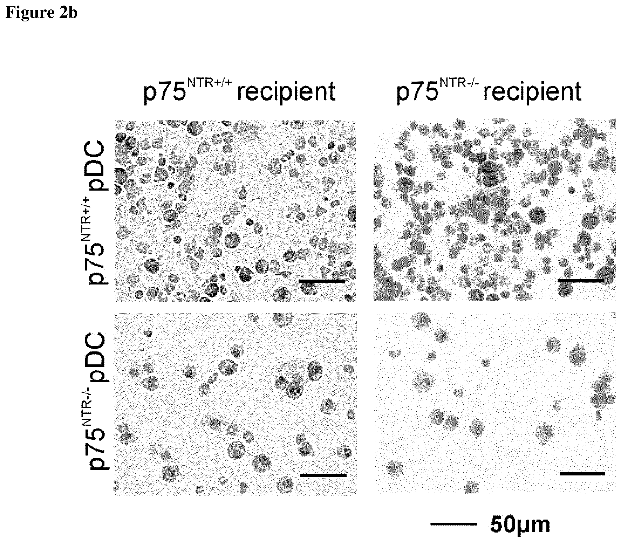

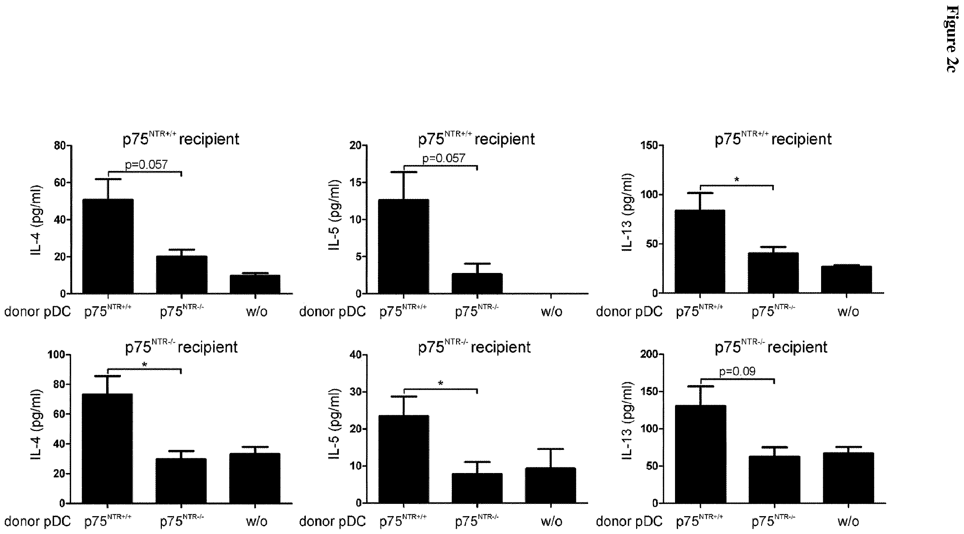

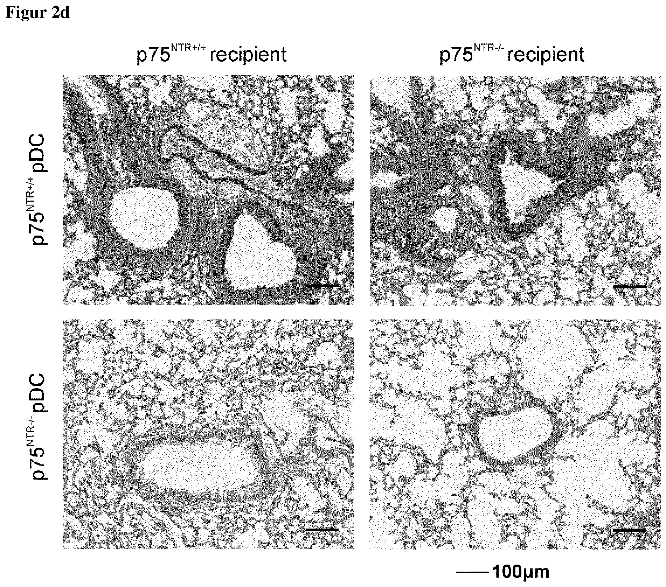

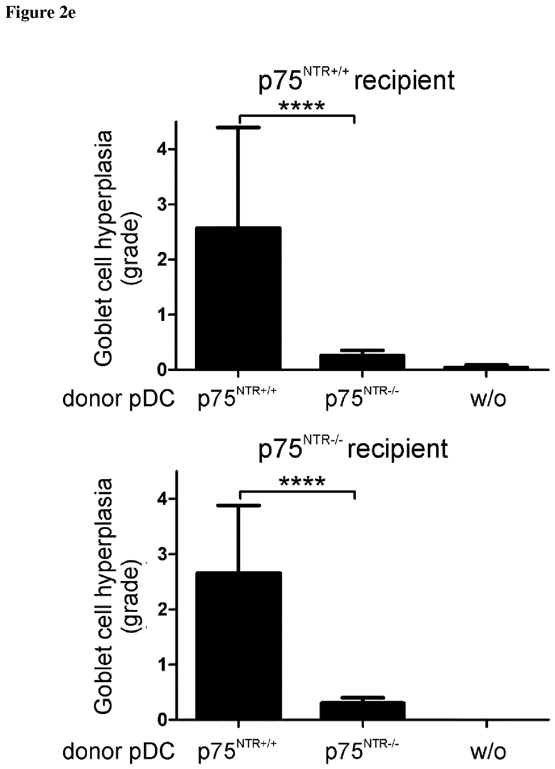

- **Figure 2**: shows the results of the investigation of the role of p75NTR expressed on murine PDCs in the process of disease triggering in a mouse model of OVA-mediated allergic asthma. After provocation with OVA aerosol characteristic symptoms of asthma like severe eosinophilia, lung inflammation and intensive mucus production were analyzed. p74^{NTR+/+} mice (wildtype) and p75^{NTR-/-} mice (knockout) treated with OVA-loaded p75^{NTR-/-} PDCs showed significantly reduced numbers of immune cells in the BALF (lymphocytes and eosinophils) compared to mice that received p75^{NTR+/+} PDCs (Fig. 2a, b). OVA-mediated immune response further lead to increased Th2 cytokine secretion (IL-4, IL-5 and IL-13) in the BALF of mice treated with p74^{NTR+/+} PDCs but not in mice that received p75^{NTR-/-} PDCs (Fig. 2c). Perivascular inflammation and Goblet cell hyperplasia in the lung were diminished in mice treated with p75^{NTR-/-} PDCs compared to mice treated with p75^{NTR+/+} PDCs (Fig. 2d, e).

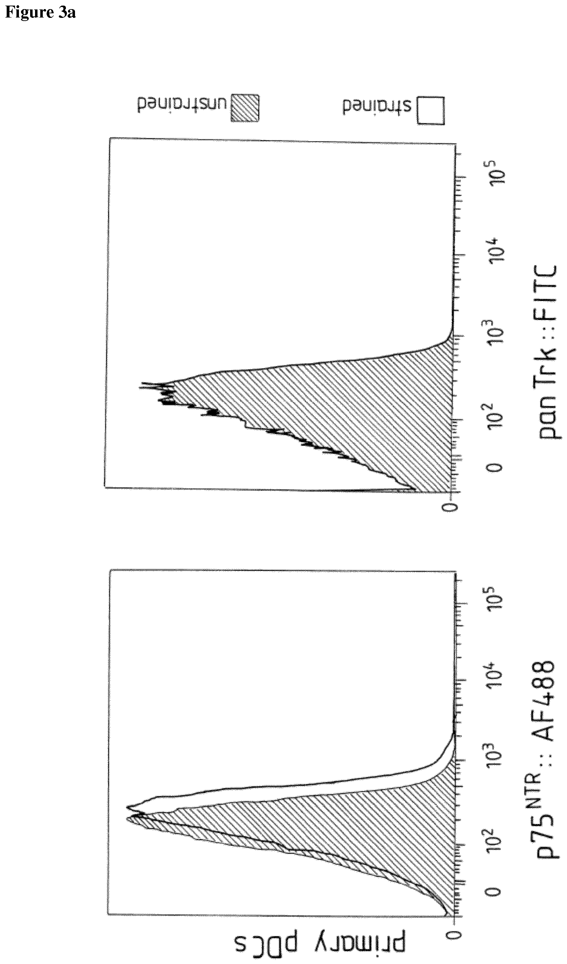

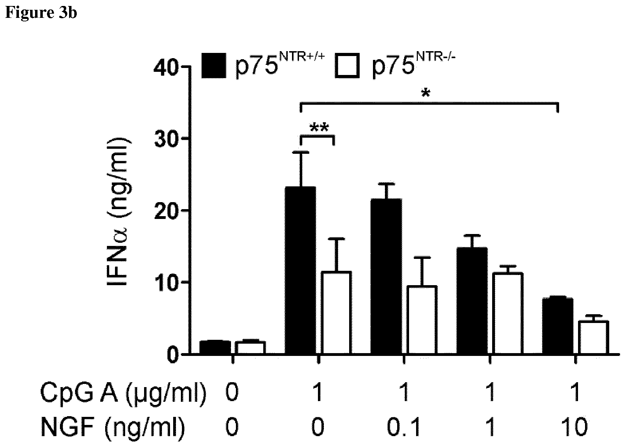

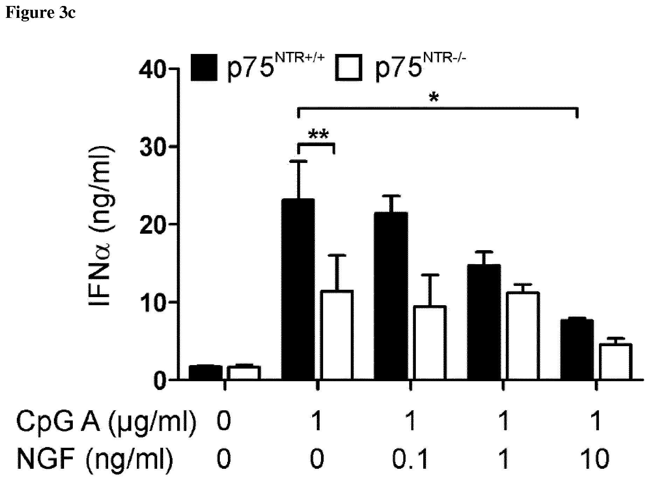

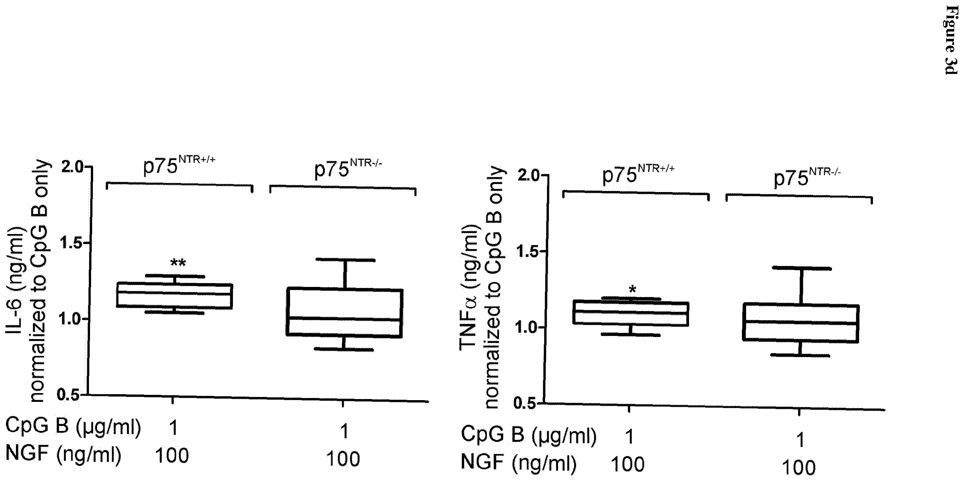

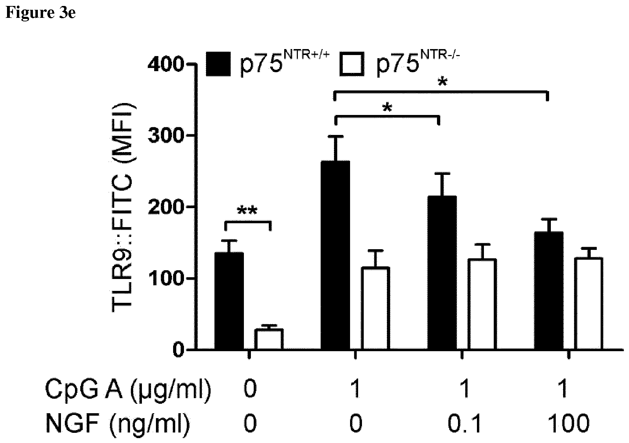

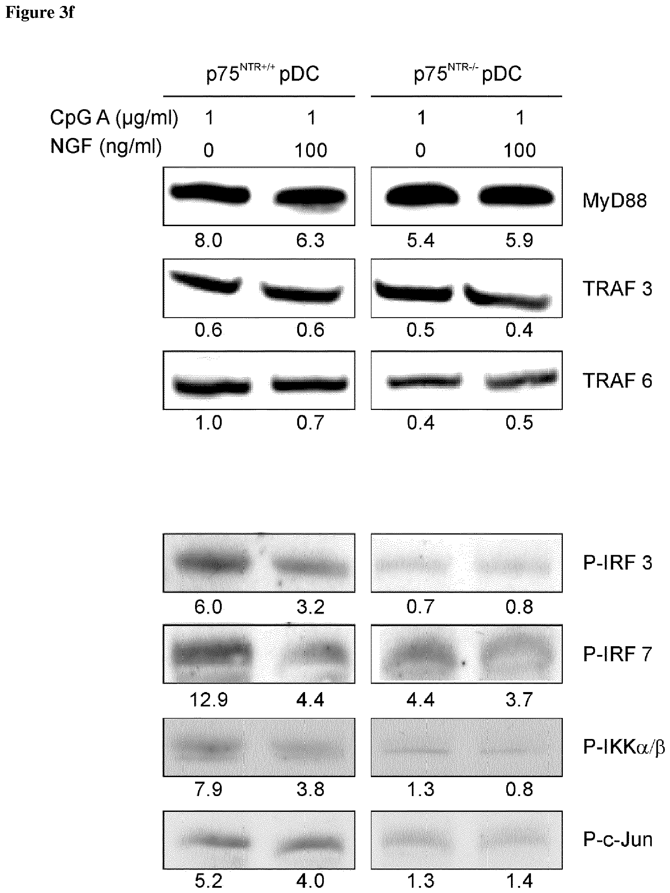

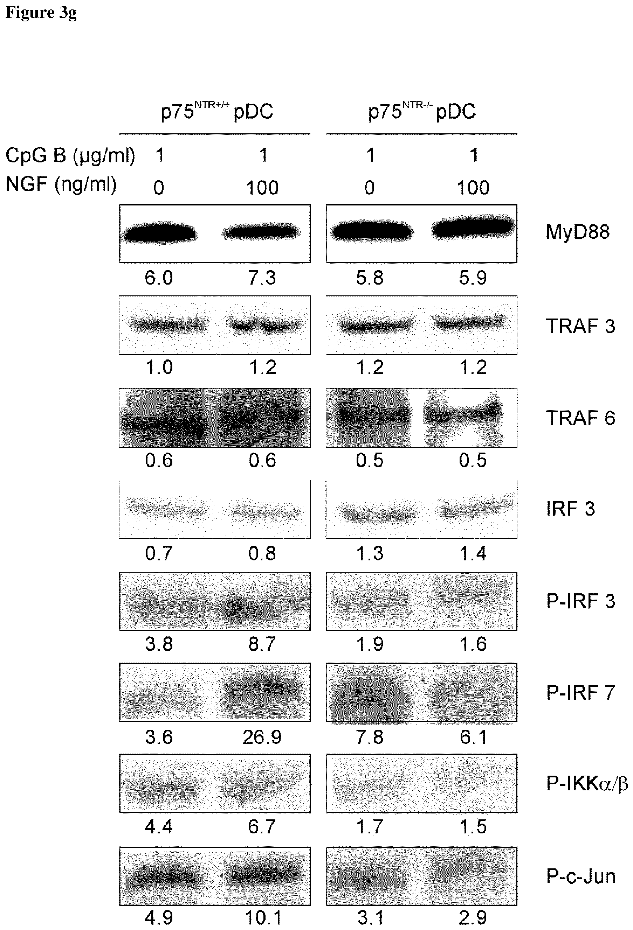

- **Figure 3**: shows the results of the investigation of the role of p75NTR expressed on murine PDCs in the process of CPG oligodeoxynucleotide stimulated immune response in vitro. Murine PDCs from the p75^{NTR+/+} (wildtype) strain express the low affinity neurotrophin receptor p75NTR, whereas the p75^{NTR-/-} (knockout) strain does not (Fig. 3a,b). The p74^{NTR+/+} PDCs do not express any of the other neurotrophin Trk receptors (Fig. 3a; with antibody staining: continuous line; without antibody staining: hatched area). In contrast to p75^{NTR-/-} PDCs, CPG A induced IFNα secretion of p75^{NTR+/+} PDCs was reduced upon addition of NGF in a concentration dependent manner, illustration a reduction of Th1 response (Fig. 3c). p75NTR+/+ PDCs secreted significantly higher amounts of pro-inflammatory cytokines IL-6 and TNFα, after stimulation with the Th2 inducing oligodeoxynucleotide CPG B (Fig. 3d) Also expression of the Toll-like receptor TLR9 expressed on PDCs was negatively influenced by NGF addition to CPG A stimulated p75^{NTR+/+} PDCs, whereas p75^{NTR-/-} showed now difference in TLR9 expression (Fig. 3e). Addition of NGF to Th1-response inducing oligodeoxynucleotide CPG A stimulated p75^{NTR+/+} PDCs react with a reduced expression of MyD88 and TRAF6, and a reduced activation (phosphorylation) of the signaling proteins IRF-3, IRF7, IKK and c-Jun (Fig. 3f). Co-Incubation of p75^{NTR+/+} PDCs with pro-inflammatory, Th2-response inducing oligodeoxynucleotide CPG B and NGF induced increased expression of MyD88 and TRAF3. Also activation (phosphorylation) of the signaling proteins IRF3, IRF7, IKK and c-Jun was increased (Fig. 3g).

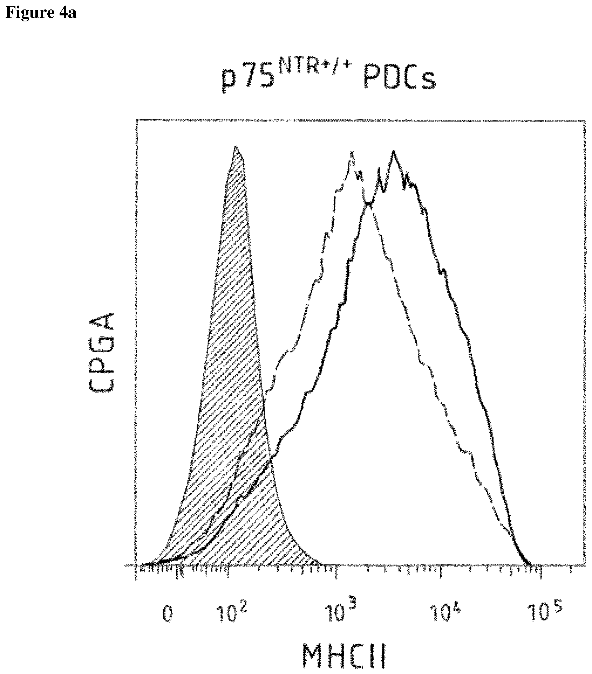

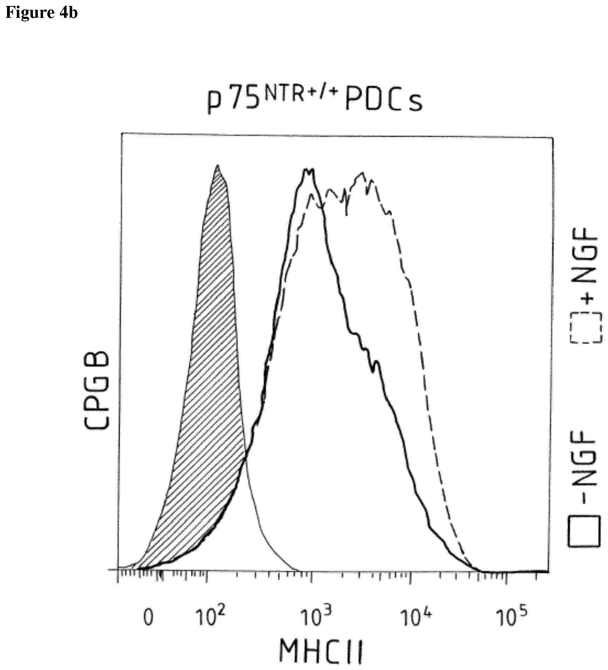

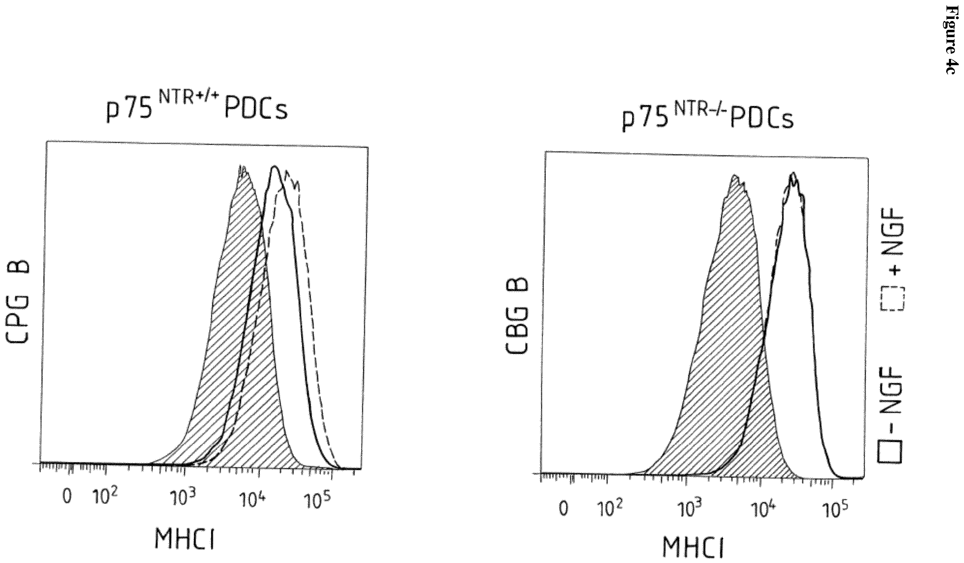

- **Figure 4**: shows the effect of NGF at the expression of Major Histocompatibility Complex proteins of Class I (MHC class I proteins) and/or of Class II (MHC Class II proteins) on murine PDCs co-stimulated with Toll-like receptor ligands CPG A and B. p75^{NTR+/+} (wildtype) PDCs react with an decreased expression of MHCII after addition of NGF to culture containing the Th1-response inducing CPG A (Fig. 4a; without NGF: continuous line, with NGF: dashed line). PDCs stimulated with Th2-response inducing CPG B showed further increase in MHCII expression upon addition of NGF to the culture (Fig. 4b; without NGF: continuous line, with NGF: dashed line). Compared to p75^{NTR-/-} (knockout) PDCs, addition of NGF to p75^{NTR+/+} PDCs lead to an further increased expression of MHCI induced by pro-inflammatory CPG B (Fig. 4c; without NGF: continuous line, with NGF: dashed line). PDCs without staining are depicted as hatched area histogram.

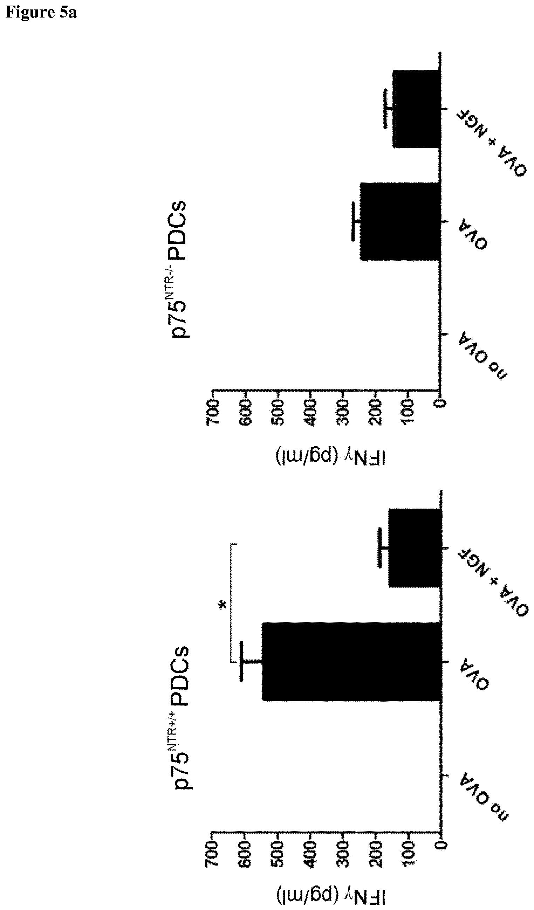

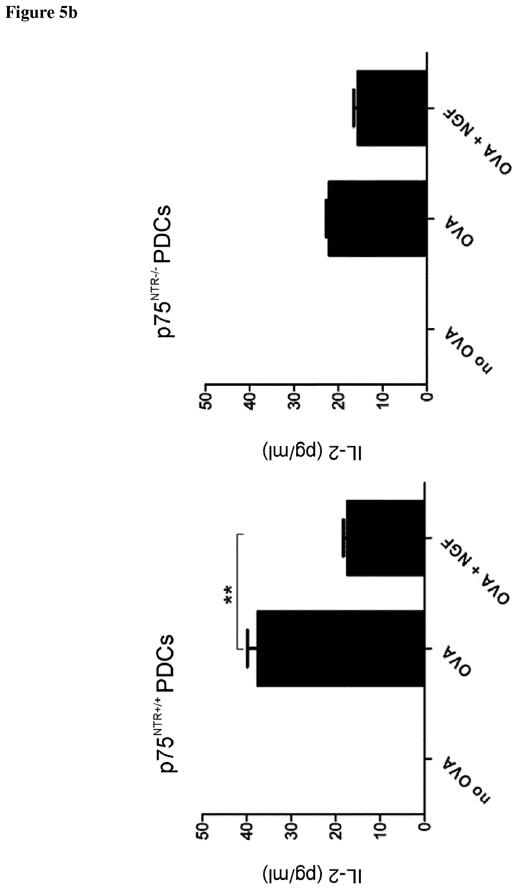

- **Figure 5**: shows the influence of NGF on the secretion of the T-cell secreted Th1 cytokines IFNγ and IL-2 in a co-culture of murine PDCs and T-cells. PDCs were isolated from either p75^{NTR+/+}(wildtype) or p75^{NTR-/-} (knockout) mouse strain. T-cells were isolated from OTII mouse strain expressing ovalbumin peptide specific T-cell receptors. In the presence of p75^{NTR+/+} PDCs presenting the ovalbumin peptide (OVA) to the T-cells, T-cells secrete the Th1 cytokines IFNγ (Fig.5a) and IL-2 (Fig. 5b). Compared to co-culture with p75^{NTR-/-} PDCs, T-cells co-cultured with PDCs from the p75^{NTR+/+} strain react with reduced secretion of both Th1 cytokines upon addition of NGF.

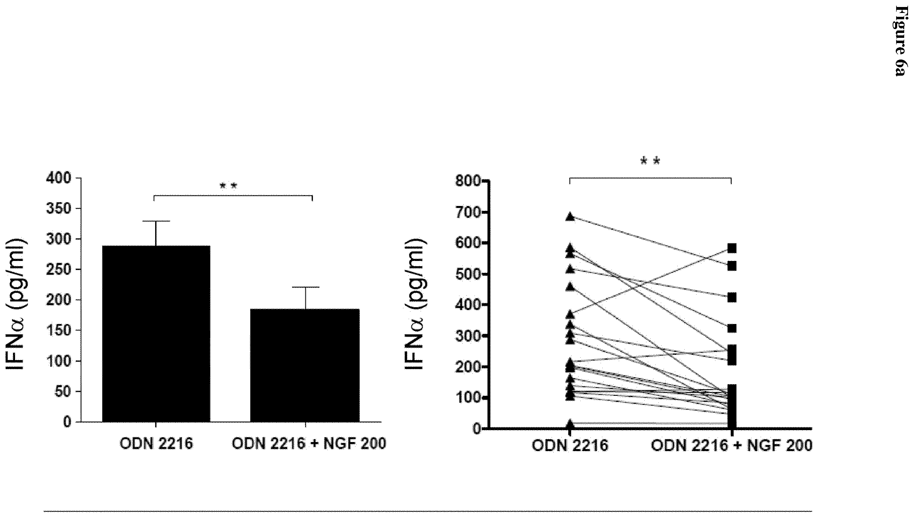

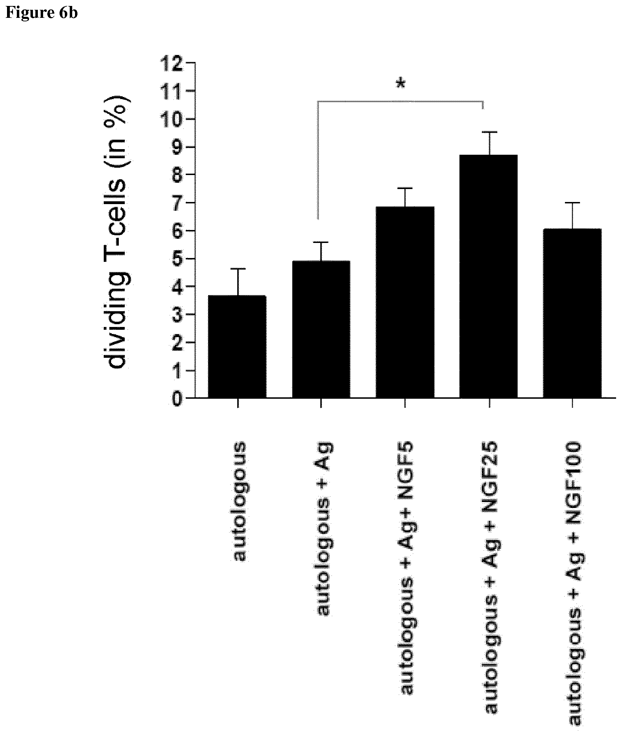

- **Figure 6**: shows graphic representations of IFNα (pg/ml) produced by human PDC activated by, ODN 2216 (▲) vs. ODN 2216 + NGF at 200 ng/ml (□) (Fig. 6a). IFNα secreted in supernatant by activated PDC was determined by ELISA. Data shown are the mean plus minus SEM (n = 20). Level of significance was chosen p < 0.05. Significant differences indicated by (p = 0.0031) and ** as determined by student's paired t-test (two- tailed). In addition, blocking of p75NTR receptor by synthetic peptide PEP5 in the presence of NGF resulted in significantly increased secretion of IFNα (Fig. 6b) Data shown are the mean plus minus SEM (n = 23) in the left panel, and (n = 9) in the right panel. Level of significance indicated by *, and ** was determined by student's paired T-test (two tailed); ns = non-significant

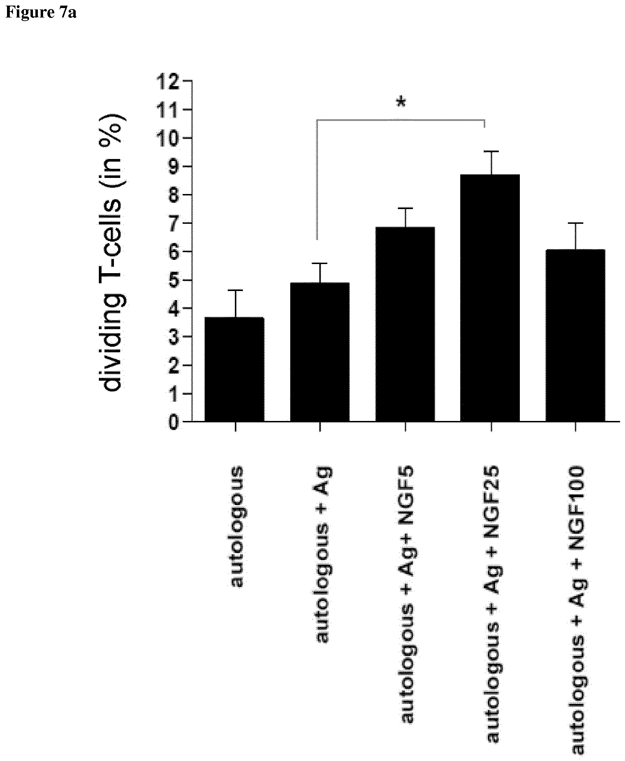

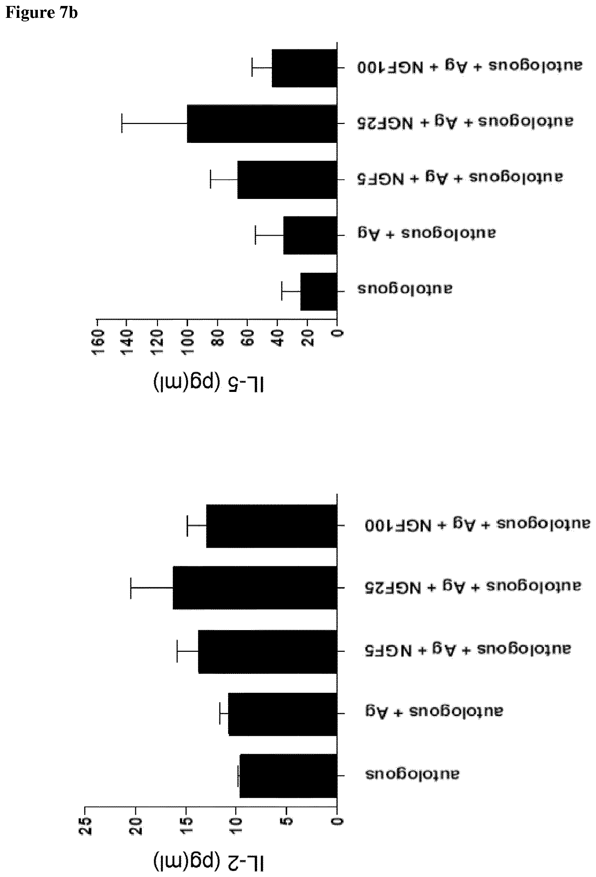

- **Figure 7**: shows the influence of NGF on the proliferation of T-cells and the secretion of pro-inflammatory cytokines in a co-culture of T-cells and PDCs isolated from allergic patients. Upon addition of NGF to the co-culture, T-cells showed an increased proliferation in the presence of specific allergen (Fig. 7a). T-cells also react with an increasing secretion of pro-inflammatory cytokines IL-2 and IL-5 (Fig. 7b). Values are shown as mean with SEM of four different allergic donors (n = 4). Values were compared using one-way ANOVA multiple comparison method (Tukey's). Differences were considered significant when p < 0. 05. Ag.: Allergen

### Detailed description of the invention

### Definitions

The term "p75NTR" herein refers to the Low-Affinity Nerve Growth Factor Receptor (also called LNGFR, p75 neurotrophin receptor, TNFRSF16 (TNFR superfamily, Member 16), Gp80-LNGFR, p75, p75ICD, Member 16, CD271 or NGF receptor). "p75NTR" is one of the two receptor types for the neurotrophins, a family of protein growth factors that stimulate neuronal cells to survive and differentiate. "p75NTR" as used herein shall embrace the p75NTR protein as usually expressed in mammalian cells but also all splice variants thereof. Splice variants of p75NTR can be formed by "alternative splicing", a regulated process during gene expression that results in a single gene coding for multiple proteins. During the process of alternative splicing, particular exons of a gene may be included within or excluded from the finally processed messenger RNA (mRNA), which is produced from that gene. Consequently the proteins translated from alternatively spliced mRNAs will contain differences in their amino acid sequence and, often, in their structure. Preferably, in accordance with the present invention, the p75NTR protein is encoded by the gene having the nucleic acid sequence of SEQ ID No. 4 (Gene ID 4804; NCBI reference sequence NM_002507.3). Most preferably, the p75NTR protein as used herein has the amino acid sequence of SEQ ID No. 3 (Swiss-Prot Accession No. P08138.1).

"Activation," "stimulation," and "treatment," as it applies to cells or to receptors, may have the same meaning, e.g., activation, stimulation, or treatment of a cell or receptor with a ligand, agonist or antagonist unless indicated otherwise by the context or explicitly.

"Activation" can refer to cell activation as regulated by internal mechanisms as well as by external or environmental factors.

"Ligand" encompasses natural and synthetic ligands, e.g., cytokines, cytokine variants, analogues, muteins, and binding compositions derived from antibodies. "Ligand" also encompasses small molecules, e.g., peptide mimetics of cytokines, peptide mimetics of antibodies, nucleic acids and nucleic acid mimetics.

An "agonist" is a chemical that binds to a receptor and activates the receptor to produce a biological response. Whereas an agonist causes an action, an antagonist blocks the action of the agonist and an inverse agonist causes an action opposite to that of the agonist

An "antagonist" is a ligand that blocks agonist-mediated responses upon binding to a receptor. The binding of an "antagonist" disrupts the interaction and inhibit the function of an "agonist" at receptors. "Antagonists" mediate their effects by binding to the active site or to allosteric sites on receptors, or they may interact at unique binding sites not normally involved in the biological regulation of the receptor's activity. "Antagonist activity" may be reversible or irreversible. The majority of drug antagonists achieve their potency by competing with endogenous ligands or substrates at structurally defined binding sites on receptors.

"Response," e.g., of a cell, tissue, organ, or organism, encompasses a change in biochemical or physiological behavior, e.g., concentration, density, adhesion, or migration within a biological compartment, rate of gene expression, or state of differentiation, where the change is correlated with activation, stimulation, or treatment, or with internal mechanisms such as genetic programming.

"Activity" of a molecule may describe or refer to the binding of the molecule to a ligand or to a receptor, to catalytic activity; to the ability to stimulate gene expression or cell signaling, differentiation, or maturation; to antigenic activity, to the modulation of activities of other molecules, and the like. "Activity" of a molecule may also refer to activity in modulating or maintaining cell-to-cell interactions, e.g., adhesion, or activity in maintaining a structure of a cell, e.g., cell membranes or cytoskeleton.

"Proliferative activity" encompasses an activity that promotes, that is necessary for, or that is specifically associated with, e.g., normal cell division, as well as cancer, tumors, dysplasia, cell transformation, metastasis, and angiogenesis.

"Administration" and "treatment," as it applies to treatment of a human subject, research subject, veterinary subject, animal, or cell, refers to contact of a pharmaceutical, therapeutic, diagnostic agent or composition, or placebo, to the human subject, animal, or cell. Treatment of a cell encompasses contact of a reagent to the cell, as well as contact of a reagent to a fluid, where the fluid is in contact with the cell.

"Administration" and "treatment" also encompass ex vivo treatment, e.g., ex vivo treatment to a cell, tissue, or organ, followed by contact of the cell, tissue, or organ, to the subject or animal, even where the agent or composition has been metabolized, altered, degraded, or removed, during or after the ex vivo treatment.

"Candidate compound" or "test compound" refers, e.g., to a molecule, complex of molecules, or mixture of molecules, where the candidate compound is used in the development or identification of a therapeutic or diagnostic agent. Testing or screening of a candidate compound is used to determine if the compound can be useful as therapeutic or diagnostic. "Candidate compounds" encompass, e.g., polypeptides, antibodies, natural products, synthetic chemicals, organic compounds, inorganic compounds, nucleic acids and combinations thereof with a second therapeutic or diagnostic, or a carrier, diluent, stabilizer, or excipient.

"Disorder" or "disease" refers to a pathological state, or a condition that is correlated with or predisposes to a pathological state. In particular, "disorder" or "disease" is an impairment of the normal state of the living animal or human body or one of its parts that interrupts or modifies the performance of the vital functions, is typically manifested by distinguishing signs and symptoms, and is a response to environmental factors (as malnutrition, industrial hazards, or climate), to specific infective agents (as worms, bacteria, or viruses), to inherent defects of the organism (as genetic anomalies or impaired functionality of the immune system), or to combinations of these factors.

"Infectious disorder" or "infectious diseases" refers, e.g., to a disorder resulting from a microbe, bacterium, parasite, pathogenic fungus, viruses and the like, as well as to an inappropriate, ineffective, or pathological immune response to the disorder.

"Oncogenic disorder" encompasses a cancer, transformed cell, tumor, dysplasia, angiogenesis, metastasis, and the like, as well as to an inappropriate, ineffective, or pathological immune response to the disorder.

"Effective amount" means, e.g., an amount of a p75NTR agonist, antagonist, or binding compound or composition sufficient to ameliorate a symptom or sign of a disorder, condition, or pathological state.

"Expression" refers to a measure of mRNA or polypeptide encoded by a specific gene. Units of expression may be a measure of, e.g., the number of molecules of mRNA or polypeptide/mg protein in a cell or tissue, or in a cell extract or tissue extract. The units of expression may be relative, e.g., a comparison of signal from control and experimental mammals or a comparison of signals with a reagent that is specific for the mRNA or polypeptide versus a reagent that is non-specific.

"Inflammatory disorder" or "inflammatory disease" means a disorder or pathological condition where the pathology results, in whole or in part, from an increase in the number and/or increase in activation of cells of the immune system, e.g., of T-cells, B-cells, monocytes or macrophages, alveolar macrophages, dendritic cells, NK cells, NKT cells, neutrophils, eosinophils, or mast cells.

An "immune disorder" or "immune disease" is a dysfunction of the immune system. These disorders develop either because the components of the immune system are affected, or because the immune system is overactive or underactive. Furthermore, these disorders can be congenital or acquired.

"Immunotherapy" means the treatment of a disease by inducing, enhancing, or suppressing an immune response. Immunotherapies designed to elicit or amplify an immune response are classified as activation immunotherapies, while immunotherapies that reduce or suppress are classified as suppression immunotherapies.

"Knockout" (KO) refers to the partial or complete reduction of expression of at least a portion of a polypeptide encoded by a gene, e.g., the p75NTR gene, where the gene is endogenous to a single cell, selected cells, or all of the cells of an animal such as a mammal. KO also encompasses embodiments where biological function is reduced, but where expression is not necessarily reduced, e.g., an p75NTR polypeptide comprising an expressed p75NTR polypeptide that contains an inserted inactivating peptide, oligopeptide, or polypeptide. Disruptions in a coding sequence or a regulatory sequence are encompassed by the knockout technique. The cell or mammal may be a "heterozygous knockout", where one allele of the endogenous gene has been disrupted. Alternatively, the cell or mammal may be a "homozygous knockout" where both alleles of the endogenous gene have been disrupted. "Homozygous knockout" is not intended to limit the disruption of both alleles to identical techniques or to identical outcomes at the genome. Included within the scope of this invention is a mammal in which one or both p75NTR alleles have been knocked out.

"Knock down" (KD) refers to a partial reduction of at least a portion of a polypeptide encoded by a gene, e.g., the p75NTR gene, where the gene is endogenous to a cell line, single cell, selected cells, or all of the cells of an animal such as a mammal. KD is achieved, e.g., by expression of a siRNA/shRNA.

"Transgenic" refers to a genetic change, produced by a technique of genetic engineering that is stably inherited. Transgenic methods, cells, and animals, includes genetic changes that result from use of a knockout technique, a knock-in technique or any other conventional techniques for the production of transgenics.

A "marker" relates to the phenotype of a cell, tissue, organ, animal, e.g., of a mouse, or human subject. A cell surface marker refers to a molecule that is located on the plasma membrane of a specific cell type or even a limited number of cell types. An intracellular marker refers to a molecule that is located inside the cell of specific cell type or even a limited number of cell types. They are normally used in identification of cell types. Markers are used to detect cells, e.g., during cell purification, quantitation, migration, activation, maturation, or development, and may be used for both in vitro and in vivo studies. An activation marker is a marker that is associated with cell activation.

"Sensitivity," e.g., sensitivity of a receptor to a ligand, means that binding of a ligand to the receptor results in a detectable change in the receptor, or in events or molecules specifically associated with the receptor, e.g., conformational change, phosphorylation, nature or quantity of proteins associated with the receptor, or change in genetic or protein expression mediated by or associated with the receptor.

"Soluble receptor" refers to receptors that are water-soluble and occur, e.g., in extracellular fluids, intracellular fluids, or weakly associated with a membrane. Soluble receptor further refers to receptors that are engineered to be water soluble.

"Specificity of binding," "selectivity of binding," and the like, refers to a binding interaction between a predetermined ligand and a predetermined receptor that enables one to distinguish between the predetermined ligand and other ligands, or between the predetermined receptor and other receptors. "Specifically" or "selectively" binding, when referring to a ligand/receptor, antibody/antigen, or other binding pair, indicates a binding reaction that is determinative of the presence of the protein in a heterogeneous population of proteins. Thus, under designated conditions, a specified ligand binds to a particular receptor and does not bind in a significant amount to other proteins present in the sample.

A "primary cell" is a cell that is directly derived from the human or animal body.

"CPG oligodeoxynucleotides" (or CPG ODN, short "CPG") are short single-stranded synthetic DNA molecules that contain a cytosine triphosphate deoxynucleotide followed by a guanine triphosphate deoxynucleotide.

A "gene" encompasses the coding region of a polypeptide and any regulatory sequences, e.g., promoters, operators, enhancers, introns, splice acceptor and donor sites, translational and transcriptional start and stop signals. The coding region may comprise one, continuous exon, or it may comprise more than one exon, i.e., it may be interrupted by one or more introns. A "gene" can encompass one or more open reading frames (ORF).

A "vaccine" is a biological preparation that improves immunity to a particular disease. A vaccine typically contains an ingredient that resembles a disease-causing microorganism and is often made from inactivated forms of the microorganism, its toxins or one of its surface proteins. The ingredient stimulates the body's immune system to recognize the ingredient as foreign, destroy it and memorize it for future infections. Vaccines can be prophylactic (e.g. to prevent or ameliorate the effects of a future infection by a pathogenic microorganism), or therapeutic (e.g., vaccines against cancer).

An "adjuvant" is a pharmacological and/or immunological agent that modifies the effect of other agents. Adjuvants are inorganic or organic chemical entities, macromolecules or entire cells of certain inactivated pathogenic microorganisms, which enhance the immune response to an antigen. They may be included in a vaccine to enhance the immune response to the supplied antigen in a subject, thus minimizing the amount of injected foreign material. Adjuvants can enhance the immune response to the antigen in different ways, e.g., by activation of the Toll-like receptor (TLR) signaling, by extending the presence of an antigen in the blood circulation, by improving the absorption of the antigen by the antigen presenting cells, by activating macrophages and lymphocytes and/or by enhancing the production of cytokines.

### Preferred embodiments of the invention

The present invention provides a cell based assay comprising human or animal primary cells or cell lines that express the nerve growth factor receptor p75NTR, characterized in that the effect of agonism or antagonism of p75NTR signaling on said cell or cell line is measured. In a preferred embodiment, the present invention provides a cell based assay comprising human or animal primary cells or cell lines that express the nerve growth factor receptor p75NTR and/or at least one protein selected from the group consisting of TLR4, TLR9, TLR7, TRAF3 and TRAF6 and signaling molecules of the Toll-like receptor pathway, comprising but not limited to MyD88, IRAKI to 4, IRF3, IRF7, wherein the effect of agonism or antagonism of p75NTR signaling on said cell or cell line is measured.

Suitably, the primary cells used in the assay of the invention are dendritic cells comprising but not limited to plasmacytoid dendritic cells (PDCs). Further suitably, the cell lines used in the assay of the invention are derived from plasmacytoid dendritic cells. In a preferred embodiment, there are transgenic cells or cell lines in the assay of the invention, which have been genetically modified to overexpress either p75NTR and/ or Toll like receptors TLR9 and/ or TLR7, or modified to elicit reduced expression thereof using siRNA, shRNA or morpholinos.

The invention further provides the use of the cell based assay in screening methods for substances that exert agonistic or antagonistic effects on p75NTR signaling.

In a further embodiment, the invention provides a screening method for agonists and antagonists of the p75NTR signaling.

In a preferred embodiment, the invention provides a screening method for agonists and antagonists of p75NTR signaling comprising the steps of:
- Contacting a human or animal primary cell or cell line that expresses the nerve growth factor receptor p75NTR, with a test substance under;
- Incubating said contacted human or animal primary cell or cell line for a period of time, which is sufficient for effecting p75NTR signaling;
- Determining the effect of the test substance on the primary cell or cell line;
- Comparing the effect of the test substance in the contacted primary cell or cell line with the effect in control cells; and
- Selecting a test substance that agonizes or antagonizes p75NTR signaling.

Suitably, the control cells or cell lines are primary cells, most suitably PDCs.

The step of contacting a human or animal primary cell or cell line that expresses the nerve growth factor receptor p75NTR, with a test substance, is preferably performed under conditions allowing the interaction of the test substance with the p75NTR protein. Further preferably, the step of contacting a human or animal primary cell or cell line that expresses the nerve growth factor receptor p75NTR, with a test substance, may be performed under conditions allowing the interaction of the test substance with the p75NTR protein and/or the interaction of the test substance with upstream or downstream factors in the p75NTR signaling pathway.

Control cells are preferably cells or cell lines that have not been contacted with the test agent.

More preferably, control cells are cells or cell lines that do not express p75NTR or that express p75NTR in a reduced amount. Said control cells or cell lines that do not express p75NTR or that express p75NTR in a reduced amount may optionally be contacted with the test substance.

In a further embodiment, the primary cells or cell lines are pre-activated prior to or during their use in the assay and screening methods of the invention. Suitable for use in the pre-activation of the primary cells and cell lines are agonists of TLR9 signaling. Preferred agonists of TLR9 signaling are for example bacterial DNA, Cpg-ODNs Class A, such as ODN 1585, ODN 2216, ODN 2336; CPG-ODNs Class B such as ODN BW006, ODN D-SL01 ODN 1668, ODN 1826, ODN 2006, ODN 2007 and CPG_ODNs Class C, such as ODN D-SL03, ODN 2395, ODN M362.

The test substance used in the assay and/or screening methods of the invention may be a natural p75NTR agonist, such as nerve growth factor (NGF), Brain-derived neurotrophic factor (BDNF), neurothrophin-3 (NT-3), neurothrophin-4 (NT-4) or neurotrophin-5 (NT-5) or a combination thereof.

The test substance used in the assay and/or screening methods of the invention may also be a precursor of a natural p75NTR agonist, such as pro-NGF, pro-BDNF, pro-NT-3, pro-NT-4, pro-NT5 or a combination thereof.

The method may be performed in presence or absence of a natural ligand of p75NTR, such as a p75NTR agonist or a p75NTR antagonist. If the method is performed in presence of a natural ligand of p75NTR, the method is suitably performed under conditions allowing the interaction of the substance with the p75NTR protein or the interaction of the test substance with said natural ligand of p75NTR.

The antagonistic or agonistic effect of the test substance on the p75NTR signaling in the assay and/or screening methods of the invention can be measured based on the expression analysis of cytokines. Suitable cytokines for expression analysis in the assay and/or screening methods of the invention comprise for example interferon alpha (IFNα), tumor necrosis factor alpha (TNFα), interleukin-4 (IL-4), interleukin-5 (IL-5), interleukin-6 (IL-6), interleukin-13 (IL-13) and other cytokines. Similar to the observed effects of NGF an agonist is expected to inhibit the expression of Th1 cytokine IFNα, while expression of Th2 cytokines IL-4, Il-5, Il-6, IL-13 and TNFα, is increased. Antagonists would be expected to inhibit described agonistic effects of Neurotrophins, where the Neurothrophin can be derived from autocrine production by the test cell or externally supplemented. An antagonist is expected to shift the cytokine production to a Th1 profile with strong induction of IFNα and a suppressed or reduced expression of inflammatory Th2 cytokines, e.g. IL-4, IL-5, IL-6, IL-13 and TNFα.

The antagonistic or agonistic effect of the test substance on the p75NTR signaling in the assay and/or screening methods of the invention can be further measured based on analyzing intracellular signaling cascade, for instance their proteins for expression level and their activation, e.g. phosphorylation. Suitable intracellular signaling cascades for analysis in the assay and/or screening methods of the invention comprise for example but not limited to the activation of TNF receptor associated factors 3 (TRAF3) and 6 (TRAF6), the activation of NF-kappa-B essential modulator (NEMO), the activation of IκB kinase (IKK), the activation of interferon regulatory factor 3 (IRF3) and 7 (IRF7), the activation of NF-κB (nuclear factor 'kappa-light-chain-enhancer' of activated B-cells) and the like. Similar to observed effects of NGF an agonist is expected to inhibit the activation of IRF3 and IRF7, while IKK and NF kappa (canonical and non-canonical pathways) are activated. An antagonist would inhibit described agonistic effects of Neurotrophin where the Neurothrophin can be derived from autocrine production by the test cell or externally supplemented.

The antagonistic or agonistic effect of the test substance on the p75NTR signaling in the assay and/or screening methods of the invention can be further determined based on surface marker and intracellular marker expression. Suitable surface marker for expression analysis in the assay and/or screening methods of the invention comprise for example Major Histocompatibility Complex proteins of Class I (MHC class I proteins) and/or of Class II (MHC Class II proteins), CD80, CD83, CD86, Blood dendritic cell antigen 2 (BDCA2), Blood dendritic cell antigen 4 (BDCA4), interleukin 3 receptor alpha (CD123), TLR7, TLR9 and the like. Based on observed effects with NGF an agonist is expected to inhibit upregulation of MHC molecules on the cell surface in environments that favor Th1 reactions, in environments that favor Th2 reaction agonists are expected to increase surface expression of MHC molecules. An antagonist would inhibit described agonistic effects of Neurotrophin, where the Neurothrophin can be derived from autocrine production by the test cell or externally supplemented

The antagonistic or agonistic effect of the test substance on the p75NTR signaling in the assay and/or screening methods of the invention can be further determined based on measuring uptake, intracellular processing and presentation of external antigens. Suitable external antigens for analysis in the assay and/or screening methods of the invention comprise for example CPG oligodeoxynucleotides, imiquod, ovalbumin, virus preparations, bacterial preparations, artificial peptide or protein purifications and the like. An agonist leads to increased uptake of external antigens by PDCs and an increased antigen epitope presentation on class I and class II major histocompatibility complex (MHC) molecules to effector cells, resulting in an intensified effector cell response. An antagonist leads to reduced antigen uptake and presentation, therefor limiting effector cell response.

The primary cells or cell lines that express p75NTR and which are used in the assay and/or screening methods of the invention, may also be co-incubated with other cells that play a central role in cell-mediated immune responses. Preferred for use in said co-incubation are T-cells.

In a preferred embodiment, the invention provides a screening method for agonists and antagonists of the p75NTR signaling comprising the steps of:
- Contacting a human or animal primary cell or cell line that expresses the nerve growth factor receptor p75NTR, which are co-incubated with T-cells, with a test substance ;
- Incubating said contacted co-culture of said human or animal primary cell or cell line and said T-cells for a period of time, which is sufficient for effecting p75NTR signaling;
- Determining the effect of the test substance on the primary cell or cell line and/or on the T-cells;
- Comparing of the effect of the test substance in the contacted primary cell or cell line and/or T-cells with control cells; and
- Selecting a test substance that agonizes or antagonizes p75NTR signaling.

The method may be performed in presence or absence of a natural ligand of p75NTR, such as a p75NTR agonist or a p75NTR antagonist. If the method is performed in presence of a natural ligand of p75NTR, the method is suitably performed under conditions allowing the interaction of the substance with the p75NTR protein or the interaction of the test substance with said natural ligand of p75NTR.

The step of contacting a human or animal primary cell or cell line that expresses the nerve growth factor receptor p75NTR, with a test substance, is preferably performed under conditions allowing the interaction of the test substance with the p75NTR protein. Further preferably, the step of contacting a human or animal primary cell or cell line that expresses the nerve growth factor receptor p75NTR, with a test substance, may be performed under conditions allowing the interaction of the test substance with the p75NTR protein and/or the interaction of the test substance with upstream or downstream factors in the p75NTR signaling pathway.

Control cells are preferably cells or cell lines that have not been contacted with the test agent.

In a preferred embodiment, control cells are used which do not naturally express p75NTR. This allows that the effect of the test substance on the p75NTR signaling can be directly and unambiguously attributed to the target p75NTR. Moreover, possible side effects of the test substance on other targets can be recognized. Accordingly, test agents that show an agonistic or antagonistic effect on the p75NTR signaling with high specificity and without unwanted side effects can be screened and selected for further development.

In a further embodiment, plasmacytoid dendritic cells in which p75NTR is knocked out or knocked down, are used as control cells. Likewise, the level of p75NTR in the control cells can be reduced otherwise. Further suitable according to the invention is the use of plasmacytoid dendritic cells, in which the expression of p75NTR is reduced or inhibited, as control cells.

Said control cells or cell lines that do not naturally express p75NTR or in which p75NTR is knocked out or knocked down may optionally be contacted with the test substance.

The antagonistic or agonistic effect of the test substance on the p75NTR signaling in the assay and/or screening methods of the invention can, alone or in addition to the aforementioned parameters, be further determined based on the stimulation of the co-incubated T-cells. T-cell activation can suitably be detected by determining T-cell cytokine expression such as for example chemokines, interferons, interleukins, lymphokines and tumour necrosis factor; T-cell proliferation; induction of antigen specific T-cell clones and/or induction of regulatory T-cells.

T-cell proliferation can be measured as described herein in example 4.

Induction of antigen specific T-cell clones can be measured by their specific cytokine secretion or their proliferation. Upon contact to antigens i.e. in co-cultures with antigen presenting cells (i.e. dendritic cells) T-cell clones secrete a pattern of cytokines. Specific cytokines for a Th1 response are IFNγ and IL-2, for Th2 IL-4, IL-5 and IL-13, for Th17 IL-17, IL-21 and IL-22 and for regulatory T-cells IL-9, IL-10 and TGFβ. T-cell cytokine secretion can be measured with ELISA, cytometric bead arrays (CBA) or ELISPOT analysis. Proliferation of T-cells can be measured by intensity quantification of fluorescent dye incorporated by T-cells (i.e. CFSE) and his intensity loss during T-cell proliferation using flow cytometry.

Induction of regulatory T-cells can be determined by co-culture assays with PDCs (Gehrie et al., Methods Mol Biol, 2011). In brief, naive T-cells were isolated from mouse spleen or human peripheral blood via magnetic bead separation (CD4+CD25-). T-cells were co-cultured with PDCs in the presence of anti-CD3 mAb (150 ng/mL), 10 ng/mL IL-2, and 10 ng/mL TGFβ. After 96 hours T-cells were stained with antibodies against CD4, CD25 and FoxP3 (intracellular) to determine the number of activated regulatory T-cells. In parallel, cytokine secretion (i.e. IL-10) of regulatory T-cells can be measured in the supernatant by ELISA.

To investigate the antagonistic or agonistic effect of the test substance on the p75NTR signaling in vivo, the primary cells or cell lines, which express p75NTR and/or at least one of TLR4, TLR7 and/or TLR9 may be administered to animal models. The test substance may be administered to the same animals prior to, together with or after administration of the primary cells or cell lines. Moreover, the primary cells or cell lines may also be pre-incubated with the test substance *in vitro* prior to administration to the animal models.

In a further embodiment of the present invention, the primary cells or cell lines, which express p75NTR and/or at least one of TLR4, TLR7 and/or TLR9, may be used *in vivo,* i.e. administered to animal models, which are specific for immune, inflammatory or proliferative diseases. Suitable animal models are selected from, for example, from OVA induced allergic asthma models, other models of allergic diseases, EAE models in mouse or rat, diabetes models, SLE models, transplantation models, GVHD models, tumor models and the like.

Suitable allergic asthma models are for example BALB/c and C57BL/6 mice. BALB/c mice typically mount Th2-dominated immune responses, and the induction of parameters of allergic responses such as allergen-specific IgE, airway hyperresponsiveness (AHR), and eosinophilic airway inflammation are robust. Conversely, C57BL/6 mice exhibit Th1-dominated immune responses, and have limitations in the development of allergic airway responses compared with BALB/c mice especially in the development of allergen-specific IgE responses and airway responsiveness to inhaled methacholine. Surprisingly, in response to allergen challenge, for example to ovalbumin (OVA), they do develop a robust BAL eosinophilic response, and in the tissue tend to accumulate more eosinophils in the parenchyma than around the airways, in contrast to BALB/c mice where eosinophils accumulate around the airways.

Experimental autoimmune encephalomyelitis (EAE) is the most commonly used experimental model for the human inflammatory demyelinating disease, multiple sclerosis (MS). EAE is a complex condition in which the interaction between a variety of immunopathological and neuropathological mechanisms leads to an approximation of the key pathological features of MS: inflammation, demyelination, axonal loss and gliosis. The counter-regulatory mechanisms of resolution of inflammation and remyelination also occur in EAE, which, therefore can also serve as a model for these processes. Well known in vivo EAE models are for example the C57BL/6 mouse, where immunization with MOG35-55 in complete Freund adjuvant (CFA) can induce monophasic or a chronic, sustained form of EAE. The former is characterized by multifocal, confluent areas of mononuclear inflammatory infiltration and demyelination in the peripheral white matter of the spinal cord. Macrophages and CD4+ T-cells are the main cell types in the inflammatory infiltrate. Other EAE models are SJL/J mice (immunization with PLP139-151), the Lewis rat (active and passive EAE induced by myelin basic protein (MBP) or transfer of MBP-specific T-cells), and the Dark Agouti (DA) rat (syngeneic spinal cord tissue or recombinant rat MOG can be used to induce EAE).

Of particular interest for the present invention are animal models of immune mediated diabetes (Type 1A). Spontaneous type 1 diabetes-susceptible models include the non-obese diabetic (NOD) mouse, the BioBreeding Diabetes-Prone (BB-DP) rat, the Komeda Diabetes-Prone (KDP) sub-line of the Long-Evans Tokushima Lean rat Lew.1.WR1 and the Lew.1AR1/Ztm rat. Multiple experimentally-induced models of type 1 diabetes are available including: 1) T-cell receptor (TCR) transgenic (Tg) and retrogenic mice with the T-cell receptors of naturally occurring diabetogenic clones 2) Neo-antigen (Ag) expression under the control of the rat insulin promoter (RIP) to establish neo-self antigen pancreatic expression that can be the target of autoimmunity, and 3) RIP-driven expression of costimulatory molecules on beta cells. Mice with knockouts of putative islet autoantigens have allowed direct testing of the pathogenic significance of specific target molecules. Strains of mice with mutations of genes associated with type 1 diabetes in man (FoxP3 and AIRE) are being studied (including an autosomal dominant "human" AIRE mutation).

Systemic lupus erythematosus (SLE) is an autoimmune disease that affects multiple organ systems. SLE is characterized by the loss of B and T-cell tolerance to one or more self-antigens, resulting in polysystemic inflammation. The most commonly used mouse strains that develop spontaneous disease include the F1 cross between New Zealand Black and New Zealand White (NZB/W) mice, MRL/lpr mice, and BXSB/Yaa mice. The common immunological and clinical manifestations of SLE in these 3 strains include hyperactive B and T-cells (their presence and interactions with each other are required for disease), high titers of several autoantibodies directed against nuclear antigens, defective clearance of immune complexes, and fatal immune glomerulonephritis.

The limiting factor for successful hematopoietic stem cell transplantation (HSCT) is graft-versus-host disease (GvHD), a post-transplant disorder that results from immune-mediated attack at the recipient tissue by donor T-cells contained in the transplant. The sequence of events that lead to the development of GvHD has largely been defined using mouse models. Early work established that T-cell alloreactivity is the underlying cause of the disease (Korngold and Sprent, 1978; Sprent et al., 1986). The pathology of both acute and chronic mouse models of GvHD relies on T-cell alloreactivity, but each form has a different phenotype owing to differential involvement of cytotoxic (CD8+) or helper (CD4+) T-cell subsets. Donor CD8+ T-cells are activated when their T-cell receptor (TCR) binds to recipient peptides presented in the context of recipient class I major histocompatibility complex (MHC) molecules. Suitable in vivo mouse models are reviewed in Schroeder M.A. & DiPersio J.F., Mouse models of graft-versus-host disease: advances and limitations; Dis Model Mech. 2011 May;4(3):318-33.

Suitable tumour models are skin cancer model (B16 melanoma) or fibrosarcoma cancer model (cell line MCA-102 or MCA-207). After injection of cancer cell lines or primary tumor cells C57BL/6 or BALB/c mice develop cancers. T-cells and DC, preferably PDCs, are incubated with tumor cell lysate *in vitro.* Afterwards T-cells alone or in combination with DCs are injected into the cancer cell bearing mouse. Efficiency of PDC immunization is measured via quantification of metastasis development and the development and activity of cytotoxic T-cells. Other tumour mouse models are, e.g., the B16-F10-induced metastatic lung cancer model (Liu et al., JCI, 2008) or the E.G7 T cell lymphoma model (Lou et al., J Immunol, 2007). In both models, activated pDCs were injected into tumour-bearing mice (tumor cells were injected before), injected before tumour cells were applied or both in parallel. After several days/weeks tumour size can be measured.

Suitable transplantation models are allogeneic organ transplantations in mouse, e.g. skin and cardiac transplantation, bone marrow transplantation, with co-transplantations of DCs preferably pDCs. For example, recipient B10.BR or BA.B10 mice were irradiated with 2 doses of 5.5 Gy separated by 3 hours on day -2. On day 0, recipient mice were transplanted with combinations of 3 to 5 × 10³ FACS-sorted HSCs, 5 × 10⁴ FACS-sorted donor pre-pDCs, and 3 × 10⁵ or 1 × 10⁶ MACS-purified T-cells from B6 CD45.1 donors. Mice were weighed twice weekly and examined daily for signs of GVHD as described previously. Moribund animals losing more than 25% of initial body weight, and mice surviving until the end of the experiment, were euthanized and tissues were processed for histopathologic analysis of tumor-trophic sites, including liver, small bowel, and large bowel. Flow cytometric chimerism analyses were performed on blood leukocytes on days 40 (± 1), 60 (± 2), and 90 (± 5) after transplant (Lu Y et al., Blood. 2012 Jan 26;119(4):1075-85). Furthermore BALB/c hearts were transplanted as fully vascularized heterotopic grafts into C57BL/6 mice as described. BALB/c cardiac grafts were transplanted by suturing of donor aorta and donor pulmonary artery end-to-side to the C57BL/6 recipient lower abdominal aorta and inferior vena cava, respectively. Recipient mice received intravenous injections in 0.5 ml PBS at various times. For tolerance, mice were treated with DST (1_107 donor splenocytes intravenously) on day -7 and 250 mg mAb to CD40L on days -7, -4, 0 and +4 (times relative to transplantation). One group received 100 mg mAb to CD40L 30 d after tolerization and mice rejected at 37-40 d. Graft function was monitored every other day by abdominal palpation. Tolerizing mice were studied at 1, 5 and 10 weeks after transplantation. Mice that had graft survival 10 weeks or more were considered 'tolerized' (called '10-week tolerized' here). Untreated control mice received hamster IgG in PBS and rejection, defined as complete cessation of a palpable beat and confirmed by direct visualization at laparotomy, occurred 1 week after transplantation (Qian S et al, Hepatology. 1994 19:916-924.

In a further preferred embodiment, the primary cells or cell lines, which express p75NTR and/or at least one of TLR4, TLR7 and/or TLR9 are pre-incubated with the test substance or p75NTR antagonists and/or agonists in the presence or absence of natural agonists of p75NTR or precursors thereof. Natural agonists of p75NTR are for example nerve growth factor (NGF), Brain-derived neurotrophic factor (BDNF), neurothrophin-3 (NT-3), neurothrophin-4 (NT-4) and neurotrophin-5 (NT-5) or a combination thereof. Precursors of natural p75NTR agonists are for example pro-NGF, pro-BDNF, pro-NT-3, pro-NT-4, pro-NT-5 or a combination thereof.

The test substance can be administered to the animal models via any suitable route. A typical administration is performed orally or intravenously.

The primary cells or cell lines, which express p75NTR and/or at least one of TLR4, TLR7 and/or TLR9, are typically injected into the blood stream or into specifically desired organs or tissues of the animal models.

In order to determine the antagonistic or agonistic effect of a test substance in vivo, T-cell activation can be detected by determining T-cell cytokine expression such as for example chemokines, interferons, interleukins, lymphokines and tumour necrosis factor; T-cell proliferation; induction of antigen specific T-cell clones and/or induction of regulatory T-cells in samples obtained from the treated animals. The samples are preferably blood samples or tissue samples.

Preferably, said sample and/or control sample has already been obtained from treated animal and/or the control animal prior to the determination of the effect of the test substance in the animal model.

The in vivo determination of the antagonistic or agonistic effect of a test substance is preferably performed in the presence of control animals. More preferably, animals of the same species and/or strain are used as control animals. Most preferably, animals which comprise at least the plasmacytoid dendritic cells but in which p75NTR is not expressed or expressed at a lower level, are used as control animals. This allows that the in vivo effect of the test substance on the p75NTR signaling can be directly and unambiguously attributed to the target p75NTR. Moreover, possible side effects of the test substance on other targets can be recognized. Accordingly, test agents that show an agonistic or antagonistic effect on the p75NTR signaling with high specificity and without unwanted side effects can be screened and selected for further development.

In one embodiment, animals which comprise at least the plasmacytoid dendritic cells but in which p75NTR are knocked out are used as control animals.

Likewise, the level of p75^{NTR} can be reduced otherwise. Further suitable according to the invention is the use of animals, which comprise at least the plasmacytoid dendritic cells but in which the expression of p75NTR is reduced or inhibited, as control animals.

In order to provide appropriate control animals, in one embodiment of the invention, plasmacytoid dendritic cells are administered to the control animals, which do not naturally express p75NTR, or in which the p75NTR gene is knocked out or in which the expression of the p75NTR gene is reduced or inhibited. In another embodiment, the p75NTR gene may be knocked out or the expression of the p75NTR gene may be reduced or inhibited not only in the administered plasmacytoid dendritic cells but also in the endogenous cells of the control animals.

Reduction or inhibition of p75NTR can be achieved e.g. using shRNA, siRNA, antisense nucleotides and the like.

A small hairpin RNA or short hairpin RNA (shRNA) is a sequence of RNA that makes a tight hairpin turn that can be used to silence target gene expression via RNA interference (RNAi). Expression of shRNA in cells is typically accomplished by delivery of plasmids or through viral or bacterial vectors. Small interfering RNA (siRNA), sometimes known as short interfering RNA or silencing RNA, is a class of double-stranded RNA molecules of about 20-25 base pairs in length. siRNA interferes with the expression of specific genes with complementary nucleotide sequences. siRNA functions by causing mRNA to be broken down after transcription resulting in no translation.

In a preferred embodiment, the invention provides a method for ex vivo determination of the antagonistic or agonistic effect of a test substance in samples which were obtained from the aforesaid animal models and control animals after the aforesaid animal models have received the plasmacytoid dendritic cells and the test substance.

The invention further relates to antagonists and agonists of p75NTR signaling that have been identified with the assay and/or the screening methods of the present invention. Agonists and antagonists - as identified with the methods disclosed herein may include proteins, nucleic acids, carbohydrates, antibodies, or any other molecules; for example, they may include small molecules and organic compounds that bind to p75NTR by a competitive or non-competitive type mechanism. Preferred are small molecule antagonists and agonists of p75NTR.

In a further embodiment, the invention provides a pharmaceutical composition comprising at least one antagonist or agonist of pNTR75 and at least one pharmaceutically acceptable carrier or excipient.

Pharmaceutical compositions suitable for use in this aspect of the invention include compositions wherein the active ingredients are contained in an effective amount to achieve the intended purpose relating to one of the diseases. The determination of a therapeutically effective dose is well within the capability of those skilled in the art and can be estimated initially either in cell culture assays, e. g. of neoplastic cells, or in animal models, usually mice, rats, rabbits, dogs, monkeys or pigs. An animal model may also be used to determine the appropriate concentration range and route of administration. This information is then commonly used to determine useful doses and routes for administration in humans.

A therapeutically effective dose refers to that amount of active ingredient, e.g., an antibody against p75NTR, or an agonist, antagonist or inhibitor of p75NTR, which ameliorates particular symptoms or conditions of the disease. For example, the amount to be administered may be effective to inhibit the activity of the p75NTR. Therapeutic efficacy and toxicity may likewise be determined by standard pharmaceutical procedures in cell cultures or with experimental animals, such as by calculating the ED50 (the dose therapeutically effective in 50% of the population) or LD50 (the dose lethal to 50% of the population) statistics. The dose ratio of toxic to therapeutic effects is the therapeutic index, and it can be expressed as the LD50/ED50 ratio. Pharmaceutical compositions, which exhibit large therapeutic indices, are preferred. The data obtained from cell culture assays and animal studies are used in formulating a range of dosage for human use. The dosage contained in such compositions is preferably within a range of circulating concentrations that include the ED50 with little or no toxicity. The dosage varies within this range depending upon the dosage form employed, the sensitivity of the patient, and the route of administration.

An exact dosage will normally be determined by the medical practitioner in light of factors related to the subject requiring treatment, with dosage and administration being adjusted to provide a sufficient level of the active moiety or to maintain a desired effect. Factors to be taken into account include the severity of the disease state, the general health of the subject, the age, weight, and gender of the subject, diet, time and frequency of administration, drug combination (s), reaction sensitivities, and tolerance/response to therapy. Long-acting pharmaceutical compositions may be administered every 3 to 4 days, every week, or even once every two weeks, depending on the half-life and clearance rate of the particular formulation.

In a preferred embodiment, the present invention provides a method for treating diseases or pathological conditions that are related to p75NTR signaling, preferably of immune diseases, comprising administering a pharmaceutically effective amount of a p75NTR agonist or p75NTR antagonist or of a pharmaceutical composition comprising the same to a subject in need thereof.

Likewise, the invention provides the use of a p75NTR agonist or p75NTR antagonist or of a pharmaceutical composition comprising the same in such methods of treatment.

Moreover, p75NTR agonists or p75NTR antagonists or pharmaceutical compositions comprising the same are provided for use in the treatment of diseases or pathological conditions that are related to p75NTR signaling.

In a further preferred embodiment, the disease or pathological condition that is related to the p75NTR signaling, is selected from the group consisting of central and peripheral neurodegenerative diseases, senile dementia, epilepsy, Alzheimer's disease, Parkinson's disease, Huntington's disease, Down's syndrome, prion diseases, amnesia, schizophrenia, depression, bipolar disorder, amyotrophic lateral sclerosis, multiple sclerosis, cardiovascular conditions, post-ischemic cardiac damage, cardiomyopathies, myocardial infarction, heart failure, cardiac ischemia, cerebral infarction, peripheral neuropathies, damage to the optic nerve and/or to the retina, retinal pigment degeneration, glaucoma, retinal ischemia, macular degeneration, spinal cord traumas, cranial traumas, atherosclerosis, stenosis, wound healing disorders, alopecia, any type of cancer, any type of tumors, any type of metastases, any type of leukemia, respiratory disorders, pulmonary inflammation, allergy, anaphylaxis, asthma, atopic dermatitis, chronic obstructive pulmonary disease, cutaneous pain, somatic pain, visceral pain, neurological pain, chronic neuropathic pain, inflammatory pain, autoimmune diseases, rheumatoid arthritis (polyarthritis, oligoarthritis), ankylosing spondylitis, collagenosis, systemic lupus erythematodes (SLE), SHARP syndrome, Sjögren's syndrome, scleroderma, polymyositis, dermatomyositis, progressive systemic sclerosis, spondyloarthritis (Morbus Bechterew, reactive arthritis, enteropathic arthritis, psoriatic arthritis, undifferentiated spondyloarthritis), rheumatic fever, Aicardi-Goutières syndrome, vasculitis, Wegener's granulomatosis disease, nephritis, stroke, ulcerative colitis, Crohn's diesease, Morbus Whipple, scleroderma, Still's disease, bronchopulmonary dysplasia (BPD), bronchiolitis, RSV-associated bronchiolitis, Diabetes mellitus, fibromyalgia syndrome, coeliac disease, Hashimoto's disease, hypothyroidism, hyperthyroidism, Addison's disease, graft versus host disease (GVHD), autoimmune thrombocytopenia, autoimmune hemolytic anemia, Löfgren syndrome, Behcet disease, nephrotic syndrome, uveitis, psoriatic arthritis, psoriasis (plaque psoriasis, pustular psoriasis), bone fractures, bone diseases, osteoporosis and all bacterial, fungal, viral infectious diseases, as well infections with eukaryotic parasites.

In a further embodiment, the present invention provides a combination of at least one compound selected from an agonist of p75NTR signaling or an antagonist of p75NTR signaling and an activator of a dendritic cell, preferably a plasmacytoid dendritic cell.

The invention further provides a pharmaceutical composition comprising said combination of at least one compound selected from an agonist of p75NTR signaling or an antagonist of p75NTR signaling and an activator of a dendritic cell, preferably a plasmacytoid dendritic cell and at least one pharmaceutically acceptable carrier or excipient.

The pharmaceutical composition comprising said combination is preferably a vaccine composition.

Said activator of the dendritic cell, preferably the plasmacytoid dendritic cell is preferably a TLR receptor agonist, most preferably a TLR9 agonist.

The combination of at least one compound selected from an agonist of p75NTR signaling or an antagonist of p75NTR signaling and an activator of a dendritic cell, preferably a plasmacytoid dendritic cell, and the pharmaceutical composition comprising said combination are especially suitable for use in immunotherapy, such as the treatment of cancer and infectious diseases. More preferably, said combination or pharmaceutical composition comprising said combination is suitable for use in the treatment of allergic diseases or in allergic desensibilization. Even preferably, said combination or pharmaceutical composition comprising said combination is suitable for use in the treatment of autoimmune diseases, chronic inflammatory diseases, GVHD or after transplantation to avoid graft failure.

In a further preferred embodiment antagonists or agonists of p75NTR signaling may be used to induce conditions comprising, but not limited to graft versus leukemia effect (GVL). GVL or graft versus tumor effect (GVT) is the beneficial aspect of the graft-versus-host disease. GVL is mainly beneficial in diseases with slow progress, e.g. chronic leukemia, low-grade lymphoma, and some cases multiple myeloma.

In a further preferred embodiment, the invention provides a method of monitoring efficacy of the therapy diseases or pathological conditions that are related to p75NTR signaling in a subject comprising the following steps:
- measuring T-cell activation such as T-cell cytokine expression, T-cell proliferation, induction of antigen specific T-cell clones, induction of cytotoxic T-cells and/or induction of regulatory T-cells in samples taken on two or more occasions from the subject; and
- comparing the level of T-cell cytokines, proliferated T-cells, antigen specific T-cell clones, induction of cytotoxic T-cells and/or regulatory T-cells in a sample taken from the subject with the level present in a sample taken from the subject prior to commencement of a therapy, and/or a sample taken from the subject at an earlier stage of a therapy.

Samples can be taken at intervals over the remaining life, or a part thereof, of a subject. i.e. the biological samples for monitoring the efficacy of a therapy can be taken on two or more occasions. Suitably, the time elapsed between taking samples from a subject undergoing diagnosis or monitoring will be 3 days, 5 days, a week, two weeks, a month, 2 months, 3 months, 6 or 12 months. Samples may be taken prior to and/or during and/or following an antiproliferative disease therapy, such as a chemotherapy. In a preferred embodiment, the method of monitoring comprises detecting a change in the amount of T-cell cytokines, proliferated T-cells, antigen specific T-cell clones, induction of cytotoxic T-cells and/or regulatory T-cells in samples taken on two or more occasions.

P75NTR on DCs, most preferably PDCs seems to function as a master switch in the regulation of immune responses. The modulation of immune responses is the major function of vaccine adjuvants. Therefore agonists and antagonists of p75NTR provide a means for novel adjuvants.

Activation of p75NTR on DCs, most preferably PDCs strongly induce Th2 immune responses. Therefore agonists can boost immunization responses in Th2 dependent vaccines. The directed immune response is similar to aluminum salts but works without inducing local inflammation. P75NTR agonists might be used to replace current vaccine adjuvants or could be used in combination to further boost a vaccine response.

In a further embodiment, the present invention thus relates to a vaccine composition comprising a modulator of p75NTR signaling, i.e. an agonist or antagonist of p75NTR signaling. Preferably, p75NTR signaling is modulated in p75NTR expressing dendritic cells, most preferably in p75NTR expressing plasmacytoid dendritic cells.

In a preferred embodiment, the invention provides the use of a vaccine composition comprising a p75NTR agonist for modulating immune responses comprising but not limited to stimulation of Th2 immune responses, suppression of Th1 immune responses, suppression of Th17 immune responses, suppression of regulatory T-cell induced tolerance and the like.

Preferred p75NTR agonists for use in the vaccine composition of the invention are selected from the group comprising NGF, BDNF, NT-3, NT-4, NT-5 and the like.

Further preferred p75NTR agonists, which are suitable for use in the vaccine composition of the invention are selected from activating antibodies, such as anti-p75NTR antibody MC192 (Kimpinski et al., Neurosci 1999, 93:253-263), activating peptides and activating small molecules (e.g. LM11A and derivative compounds, comprising but not limited to LM11A-24 or LM11A- 31, LM11A- 36) or are encoded by a nucleic acid.

In a further preferred embodiment, the invention provides the use of a vaccine composition comprising a p75NTR antagonist for modulating immune responses comprising but not limited to suppression of Th2 immune responses, stimulation of Th1 immune responses, stimulation of Th17 immune responses, suppression of regulatory T-cell induced tolerance and the like.

Preferred p75NTR antagonists for use in the vaccine composition of the invention are selected from the group comprising pro-NGF, pro-BDNF, pro-NT-3, pro-NT-4, pro-NT-5 and the like.

Further preferred p75NTR antagonists, which are suitable for use in the vaccine composition of the invention are selected from blocking antibodies (anti human p75NTR monoclonal antibody clones: ME20.4, MLR2, HB-8737, NGFR5 and derivatives and humanized versions thereof; and AB1554; antibodies that prevent binding of neurotrophins to p75NTR: Tanezumab a humanized version of MAb 911, PG110, REGN475 and Fulranumab), the blocking peptide tat-PEP5; and small molecule inhibitors (derivatives of 2-oxo-alkyl-1-piperazin-2-one; and small molecules that prevent binding of neurotrophins to p75NTR: PD 90780, ALE-054, Ro 08-2750, Y1036).

Preferred blocking peptides specifically inhibit the binding of TRAF6 to the intracellular domain of p75NTR (peptides that block the interaction of p75NTR with TRAF6 including peptides binding to the protein motif EGEKLHSDSGISVDS (SEQ ID No. 1) from the intracellular domain of p75NTR, TRAF6 decoy peptides comprising the RPTIPRNPK peptide (SEQ ID No. 2).

The vaccine composition of the invention can further comprise modulators of p75NTR signaling in combination with immune stimulating agents, which are, e.g., selected from monophosphoryl lipid A (MPL) and synthetic derivatives thereof, muramyl dipeptide (MDP) and derivatives thereof, oligodeoxynucleotides (such as CPG, etc.), double-stranded RNA (dsRNA), alternative pathogen-associated molecular patterns (PAMPs, such as E. coli heat labile enterotoxin (LT); flagellin), saponins (Quils, QS-21), small-molecule immune potentiators (SMIPs, e.g., resiquimod [R848]), cytokines, chemokines and antigens from *Mycobacterium tuberculosis* .

The vaccine composition of the invention can further comprise modulators of p75NTR signaling in combination with insoluble aluminum compounds, calcium phosphate, liposomes, microparticles (e.g., PLGA), emulsions (e.g., MF59, Montanides), virus-like particles and viral vectors.

The vaccine composition of the present invention may further comprise isolated dendritic cells, preferably isolated plasmacytoid dendritic cells, most preferably isolated p75NTR expressing dendritic cells or PDCs. In a preferred embodiment, the isolated dendritic cells are ex vivo incubated with at least one p75NTR signaling modulator prior to the administration of the vaccine composition to a subject.

In a preferred embodiment of the invention, at least one p75NTR agonist is used to prime said isolated dendritic cells to modulate immune responses comprising but not limited to stimulation of Th2 immune response, suppression of Th1 immune response, suppression of Th17 immune response and suppression of regulatory T-cell induced tolerance.

In a yet preferred embodiment of the invention, at least one p75NTR antagonist is used to prime said isolated dendritic cells to modulate immune responses comprising but not limited to suppression of Th2 immune response, stimulation of Th1 immune response, stimulation of Th17 immune response and suppression of regulatory T-cell induced tolerance.

Where the agonist or antagonist is encoded by a nucleic acid such as shRNA or siRNA, said nucleic acid is preferably transfected into the dendritic cell leading to overexpression of the agonist or antagonist in the dendritic cell.

In a further preferred embodiment, vaccine compositions comprising an agonist of p75NTR signaling selected from the group comprising NGF, BDNF, NT-3, NT-4, NT-5 or an antagonist of p75NTR signaling selected from the group comprising pro-NGF, pro-BDNF, pro-NT-3, pro-NT-4, pro-NT-5.

Examples for antagonists of p75NTR signaling, which are suitable for use in the vaccines of the invention and/or for use in therapy, preferably immunotherapy according to the invention are selected from the groups comprising:
∘ Anti human p75NTR Monoclonal antibodies, selected from clones ME20.4, MLR2, HB-8737, and NGFR5, derivatives and humanized versions of the aforementioned antibodies;
∘ The anti murine p75NTR monoclonal antibody AB1554;
∘ The Peptide tat-PEP5, peptides that block the interaction of p75NTR with TRAF6 selected from peptides binding to the protein motif EGEKLHSDSGISVDS (SEQ ID No. 1) from the intracellular domain of p75NTR, TRAF6 decoy peptides comprising the RPTIPRNPK peptide (SEQ ID No. 2);
∘ Small molecules selected from derivatives of 2-oxo-alkyl-1-piperazin-2-one, derivatives of naphthalimide
∘ siRNAs, shRNAs, morpholinos that block expression of p75NTR or downstream signaling;
∘ Neurotrophin antagonists that prevent binding of NGF or BDNF to p75NTR, selected from:
   ▪ Antibodies: derivatives and humanized versions of Tanezumab (a humanized version of MAb 911), PG110, REGN475 and fulranumab,;
   ▪ Small molecules, such as PD 90780, ALE-054, Ro 08-2750, and Y1036.

Examples for activators of a dendritic cell, preferably a plasmacytoid dendritic cell, which are suitable for use in the vaccines of the invention and/or for use in therapy, preferably immunotherapy according to the invention are selected from the groups comprising:
▪ TLR9 agonists, such as:
- Bacterial DNA;
- CPG-ODNs Class A, such as ODN 1585, ODN 2216, ODN 2336,;
- CPG-ODNs Class B, such as ODN BW006, ODN D-SL01 ODN 1668; ODN 1826, ODN 2006, ODN 2007; and
- CPG-ODNs Class C, such as ODN D-SL03, ODN 2395, ODN M362.

Examples for agonists of p75NTR signaling, which are suitable for use in the vaccines of the invention and/or for use in therapy, preferably immunotherapy according to the invention are selected from the groups comprising:
∘ Neurothrophins, selected from NGF, NGF-Delta 9/13 mutant, BDNF,NT-3, NT-4, NT-5, proNGF, proBDNF, proNT-3, proNT-4, pro-NT-5;
∘ Small molecules selected from LM11A-24 or LM11A-31;
∘ Nucleic acids coding for p75NTR, constitutively active p75NTR, or fragments thereof.

The invention is now further described in the following working examples.

### Examples of the invention

### Example 1: Peripheral human blood samples

Blood samples used for cell purification were obtained from two different sources:
a) Fresh buffy coat samples, not older than 8 hours, served as the source of plasmacytoid dendritic cell (PDC) used in oligodeoxynucleotides (ODN) and anti FcεRIα stimulation experiments.
b) CD4⁺ T helper cells and PDC used in antigen mediated autologous CD4⁺ T cell proliferation assays were purified from peripheral blood (80 ml) obtained from specified donors who are known to have allergy against certain allergens. Peripheral blood was drawn in tubes containing Li-Heparin as anti-coagulant (S-Monovette Li-Heparin 7.5 ml, Sarstedt).

### Example 2: Lymphocyte separation from peripheral blood by density gradient centrifugation

Peripheral blood was transferred to 50 ml tube and centrifuged (470g for 30 minutes at room temperature (RT). Intermediate leukocyte layer, between the sedimented erythrocytes and upper phase thrombocytes, was taken off along with few milliliters of erythrocytes. In a fresh 50 ml tube leukocytes were diluted with 3 volumes of IX PBS containing 2 mM EDTA and 0.5 % BSA (PBS E/B). The mixture was layered carefully on the top of ficoll separation solution (Percoll separation solution, density 1.074 g/ml, Biochrom AG) and centrifuged (1000 g without brakes for 20 minutes at RT). Erythrocytes and granulocytes sedimented to the bottom of the tube, mononuclear cells (lymphocytes) and platelets were collected in a fresh tube from the interface between the plasma layer in upper phase and sedimented erythrocytes/granulocytes. Collected cells were washed once with 50 ml PBS E/B and centrifuged (300g for 10 minutes at RT). Supernatant was removed completely. For the depletion of platelets, following the first wash, the mononuclear cells pellet was washed twice by adding 50 ml PBS E/B and centrifugation (200 g for 15 minutes at RT). Upon centrifugation at 200 g, most of the platelets remain in the supernatant. The supernatant was discarded. Lymphocytes pellet was re-suspended in 20 ml PBS E/B. To remove cell clumps and blood clots, that might clog the MACS cell separation columns during cell purifications, cells were passed through a nylon mash having 40 µm pore size (Cell Strainer, BD Biosciences).

### Example 3: Cell purifications

### CD4+ T helper cell purification

Mononuclear cells separated from peripheral blood were counted. CD4⁺ T helper cells were purified from peripheral blood mononuclear cells by negative selection using CD4⁺ T cell isolation kit II (Miltenyi Biotec), as per manufacturer's instructions with slight modifications. Briefly, the cell pellet was re-suspended in 50 µl PBS E/B. Then 12 µl of Biotin-labeled antibody cocktail per 10⁷ total cells were added and the cell suspension was incubated at 4 °C for 15 minutes. 50 µl of PBS E/B was added followed by addition of 25 µl of anti-biotin microbeads per 10⁷ total cells. Cells were incubated at 4 °C for another 20 minutes, followed by washing with 10 ml PBS E/B and centrifugation (470 g for 6 minutes). Supernatant was taken off completely and pellet was re-suspended in 500 µl of PBS E/B. Cell suspension was applied to equilibrated MACS MS separation column and enriched CD4⁺ T helper cells fraction was obtained in the flow through. The purity of isolated CD4⁺ T helper cells was over 95 % as assessed by flow cytometric analysis after co-staining with monoclonal mouse anti human CD3-PE (BD Biosciences) and monoclonal mouse anti human CD4-FITC (BD Biosciences). When more than 10⁷ cells were used volumes of reagents and buffer were scaled-up, accordingly.

### Plasmacytoid dendritic cell (PDC)/ BDCA4⁺ cell purification

Alike CD4⁺ T helper cells isolation from peripheral blood mononuclear cells, total cell numbers were determined prior to purification of PDC. PDC were purified by using CD304 (BDCA-4/ Neuropilin-1) microbead kit (Miltenyi Biotech) by positive selection, following manufacturer's instructions with some modifications. Briefly, the cell suspension was centrifuged (450 g for 6 minutes) and the pellet was re-suspended in 300 µl of PBS E/B. Then 100µl of each FcR blocking reagent and CD304 (BDCA-4/Neuraophilin-1) microbeads were added per 10⁸ total cells. Cell suspension was incubated at 4 °C for 20 minutes. Cells were washed with 10 ml PBS E/B and centrifuged (470 g for 6 minutes). Cell pellet was re-suspended in 500 µl of PBS E/B and was loaded on rinsed MACS LS cell separation column (Miltenyi Biotech). Labelled cells were attached to the separation column. The separation column was washed three times with 1 ml PBS E/B. To increase the purity of PDC, the eluted fraction was enriched over second MACS MS cell separation column. Magnetic cell separation procedure as described for first LS column was repeated for second MS column except that washing of the MS column was carried out with 500 µl PBS E/B. Purified PDC were counted and purity was assessed by flow cytometric analysis after staining the cells with monoclonal mouse anti human BDCA2- PE (Miltenyi Biotech) and monoclonal mouse anti human CD271-APC (Miltenyi Biotech). Volume of reagents and buffers mentioned are for up to 10⁸ total cells. Whenever, higher than given total cells numbers were used the volume of reagents and buffers were also scaled-up accordingly.

### Example 3: In vitro stimulations of PDC

### IFN-alpha produced by Oligodeoxynucleotides (ODN) stimulated PDC

PDC isolated from peripheral blood were taken up in RPMI 1640 medium (PAA) containing penicillin G (100 U/ml), streptomycin (100 mg/ml), L-glutamine (2 mM), 10 % heat-inactivated fetal calf serum (FCS) and Interleukin-3 (lL-3, R&D Systems) at 10 ng/ml. 5x10⁴ cells were seeded per well in 200 µl medium in U-shaped bottom 96- well plate and incubated at 5 % CO₂ and 37 °C. For IFNα induction, stimulatory ODN 2216 and control ODN 2243 (Alexis Biochemicals), were added at 0.33 µg/well to the designated wells. p75NTR blocking peptide TAT-Pep5 (Calbiochem) was employed at 100nM to designated wells. NGF (R&D Systems) was added at 200 ng/ml to the allocated wells. All components were added in order of succession as mentioned. After 12-14 hours stimulation the plate was centrifuged (270 g for 5 minutes). Supernatant was collected and was analyzed for IFNα quantification by ELISA (Bender MedSystems).

### IFNα ELISA

IFNα ELISA was carried out according to manufacturer's instructions with slight modifications. In short, 100 µl of 10 µg/ml coating antibody in PBS was added to each of the allocated wells on flat bottom 96 well EIA/RIA stripweIl plate (Corning Incorporated). Plate was covered with Parafilm M (Pechiney Plastic Packaging Company) and incubated over night at 4°C. Wells were aspirated and washed three times with washing buffer (PBS containing 0.05 % Tween 20). Plate was blocked by adding 250 µl assay buffer (5 g BSA added to 1 liter washing buffer) to each well and was incubated at room temperature for 2 hours. Before adding samples, the wells were emptied and plate was washed twice with 300 µl washing buffer. 100 µl assay buffer was added in duplicate to blank wells and wells allocated for standard, leaving the first wells (500 pg/ml) empty. 90 µl assay buffer was added in duplicate to all wells designated for samples. 50 ng/ml IFN-alpha standard protein was diluted in 500 µl assay buffer to obtain final concentration of 500 pg/ml. IFN-alpha row dilutions ranging from 500 to 8 pg/ml served as standard. 10 µl supernatant was added and mixed to the allocated wells. Horseradish Peroxidase (HRP)-conjugated detection antibody was diluted 1:1000 with assay buffer and 50 µl was added to all the wells, including blank wells. Plate was incubated at room temperature for 2 hours. The contents of wells were removed and wells were washed 3 times with 300 µl wash buffer per well. 100 µl 3, 3', 5, 5'-Tetramethylbenzidine (TMB) substrate solution (Sigma) was added to all wells and the plate was incubated at room temperature for 10 minutes. When dark blue colour was developed in the well with highest concentration protein standards, enzyme-substrate reaction was stopped by adding 100 µl of 4N sulfuric acid solution into each well. Absorbance of whole plate was read on spectrophotometer (TECAN, Infinite 200) at 450 nm as primary wave length and 630 nm as reference wave length.

### IL-6 produced by anti-FcεRIα activated PDC

PDC isolated from peripheral blood were taken in RPMI 1640 medium (PAA) with penicillin G (100 U/ml), streptomycin (100 mg/mi), L-glutamine (2 mM), 10 % heat inactivated fetal calf serum and IL-3 (R&D system) at 10 ng/ml. 2 x 10⁵ cells were seeded per well in 200 µl medium in U- shaped bottom 96-well plate and incubated at 5 % CO₂ and 37°C. For IL-6 generation, mouse anti human FcεRI alpha-FITC (eBioscience) was added at 250 ng/ml to designated wells. p75NTR blocking peptide TAT-Pep5 (Calbiochem) was employed at 100nM to designated wells. NGF (R&D Systems) was added at concentration of 25 ng/ml to specified wells. All components were added following the sequence mentioned After 14 hour's stimulation the plate was centrifuged (270 g for 5 minutes). Supernatant was analyzed for IL-6 by ELISA (Bender MedSystems).

### IL-6 ELISA

IL-6 ELISA was carried out following manufacturer's specifications with few modifications. In short, 100 µl of 2.5 µg/ml coating antibody in PBS was added to each of the allocated wells on flat bottom 96 well EIA/RIA stripwell plate (Corning Incorporated). Plate was covered with Parafilm M (Pechiney Plastic Packaging Company) and incubated over night at 4°C. Wells were aspirated and washed three time with 300 µl washing buffer (PBS containing 0.0005 % Tween 20) per well. Plate was blocked by adding 250 µl assay buffer (washing buffer containing 0.005 % BSA) to each well and plate was incubated at room temperature for 2 hours. Before adding samples the wells were emptied and plate was washed twice with 300 µl washing buffer. 100 µl assay buffer was added in duplicate to blank wells and wells allocated for standard. 60 µl assay buffer was added in duplicate to all the wells designated for samples. 2 ng/ml IL-6 standard proteins was diluted in 250 µl assay buffer to obtain final concentration of 200 pg/ml. Serial dilutions of IL-6 protein ranging from 100 to 1.6 pg/ml served as standard. 40 µl supernatant was added and mixed to the wells allocated for samples. Biotin-conjugated detection antibody was diluted 1:1000 with assay buffer and 50 µl was added to all the wells. Plate was incubated at room temperature for 2 hours. The contents of wells were removed and swashed 3 times with 300 µl of wash buffer per well. 100 µl of streptavidin-HRP, 1:5000 diluted with assay buffer, was added to all the wells and the plate was incubated at room temperature for an hour. Wells were aspirated and were washed 3 times with 300 µl wash buffer per well. 100µl TMB substrate solution (Sigma) was added to all wells, including blank wells and the plate was incubated in dark at room temperature for 10 minutes. Enzyme-substrate reaction was stopped by adding 100 µl of 4N sulfuric acid, into each well before positive wells were no longer properly recordable. Absorbance of whole plate was read on spectrophotometer at 450 nm as primary wave length and 620 nm as reference wave length.

### Example 4: Antigen mediated T cell proliferation assay

### Carboxyfluorescein succinimidyl ester (CFSE) labelling of CD4⁺ T helper cells

Purified CD4⁺ T helper cells (see above) were washed once with PBS. 3 - 8x10⁶ cells were re-suspended in 1 ml PBS containing 5% BSA. One aliquot of CFSE powder (Molecular Probes, Invitrogen Technologies) was dissolved in 18 µl DMSO to obtain final concentration of 5 mM. CD4⁺ T-cells were stained with a 1 µM final concentration of CFSE by incubating the cell suspension plus 1 µM CFSE at 37°C for 8 minutes. 1 ml pre-warmed FCS was added to the suspension and the cells were washed with RPMI medium twice. Cell number was determined.

### Co-culture of autologous PDC and CFSE labelled CD4⁺ T helper cells

Antigen mediated CD4⁺ T helper cell proliferation responses were assayed using purified and CFSE labelled CD4⁺ T helper cells in co-culture with purified PDC/ BDAC4⁺ cells. PDC and T-cells were re-suspended separately in RPMI-1640 medium supplemented with penicillin G (100 U/ml), streptomycin (100 mg/ml), L-glutamine (2 mM) and 10% human AB serum. The ratio of PDC to T cell in co-culture was kept 1:6. Antigens were added to co-culture at 50 SBE U/ml. NGF was added at the concentration of 5, 25 and 50 ng/ml. The assay was set up in U-bottom 96-well plate at 37°C in 5 % CO₂. After 5 days of co-culture, supernatant was analyzed for Th1/Th2 cytokines by using BD cytokine bead array (CBA) Human Th1/Th2 cytokine kit (BD Biosciences) and percentages of proliferating CFSE-low CD4⁺ T-cells were analyzed by flow cytometry.

### Cytokine measurement by cytokine bead array (CBA)

The concentrations of IL-2, IL-4, IL-5, IL-10, IFNγ and TNFα, in the supernatant were determined by using the CBA kit following manufacturer's instruction with modification in data analysis using Microsoft Excel. Briefly, a CBA comprises beads exhibiting series of discrete fluorescence intensities that is resolved in FL3 channel of a flow cytometer. Each series of beads is coated with a monoclonal antibody specific for a single cytokine, and a mixture of six beads can detect six different cytokines in a single sample. The PE-conjugated detection antibody stains the beads proportionally to the amount of cytokine bound. After fluorescence intensity calibration and color compensation procedures, standards and test sample (supernatant) were analyzed with FACS LSRII flow cytometer equipped with DIVA software (BD Biosciences). The standard curve created for each cytokine was used to calculate the cytokine concentration. The lower detection limits for IL-2, IL-4, IL-5, IL-10, TNFα, and IFNγ were 2.6 pg/ml, 2.6 pg/ml, 2.4 pg/ml, 2.8 pg/ml, 2.8 pg/ml, and 7.1 pg/ml, respectively.

### Example 5: The neurotrophin NGF strongly enhances PDC-mediated allergic asthma in mice

NGF is present in the lung and increased during inflammatory processes such as allergic asthma. To investigate the impact of NGF on PDCs during allergen-mediated immune response, p75^{NTR+/+} PDCs were incubated with ovalbumin (OVA) in the presence or absence of NGF (100ng/ml) prior intratracheal instillation to p75^{NTR+/+} mice. In the BALF, numbers of eosinophiles and lymphocytes were significantly augmented when the OVA up-take by PDCs was carried out in the presence of NGF compared to PDCs incubated with OVA alone (Fig. 1a, b). Furthermore, OVA-loaded PDCs treated with NGF caused increased production of Th2 cytokines (IL-4, IL-5 and IL-13) in the lung in comparison to PDCs pulsed with OVA in the absence of NGF (Fig. 1c). Histological lung sections from mice that received OVA-loaded PDCs showed increased perivascular inflammation and enhanced mucus production (Fig. 1d). Treatment of PDCs with NGF during OVA-uptake potentiated the inflammatory phenotype in the lung (Fig. 1d). In contrast, p75^{NTR-/-} PDCs loaded with OVA in the presence or absence of NGF were not able to induce airway inflammation (data not shown). Our data indicate that NGF triggers p75NTR-expressing PDCs into a pro-inflammatory phenotype, leading to much severe airway inflammation in the asthma model.

### Example 6: NGF regulates Interferon-alpha (IFNα) production (Th1 response) by ODN (Oligodeoxynucleotides) stimulated PDC

Human PDC express the Toll- like receptor 9 (TLR9). Stimulatory ODN 2216 (A-Class CpG ODN) are recognized by TLR9 expressed by PDC. Recognition of ODN 2216 by TLR9 activates PDC and induces anti-viral IFNα secretion (Th1 response). We stimulated human peripheral blood purified PDC with ODN 2216 plus minus NGF at 200 ng/ml. After 14 hours of stimulation the supernatant was collected and analyzed for IFNα secretion by ELISA. We have used 20 samples in the study during the months of May, June and July. Our results revealed significant reduction (p = 0.0031) in IFNα secretion by ODN 2216 plus NGF 200 activated PDC compared to PDC activated with ODN 2216 without NGF (Fig. 6a). However, NGF concentrations lower than 200 ng/ml did not yield significantly different IFN-α secretion by ODN 2216 activated PDC compared with ODN 2216 activated PDC. Control ODN 2243 did not stimulate PDC at all, thus no IFN-α was detected in supernatant.

To prove that regulation of IFNα secretion in ODN 2216 activated PDC by NGF is through CD271 and not through TrkA, we used TAT-Pep 5 (p75ntr signaling inhibitor) to rescue the NGF mediated IFNα production by ODN 2216 activated PDC. In total 23 buffy coat samples were analyzed. Statistical analysis performed on all 23 samples collectively, did not reveal significant differences in NGF induced IFN-α production by ODN 2216 activated PDC compared with ODN 2216 activated PDC without NGF. Only exception being PDC activated with ODN 2216 + NGF200 + Pep5 demonstrated significant difference (p = 0.0471) in IFN-α secretion compared with PDC activated with ODN 2216 (Fig. 6b).

However, for the samples which showed NGF dependent decreased in IFNα production, addition of TAT-Pep5 (100 nM) significantly recovered (p = 0.0168) the NGF mediated IFNα secretion in ODN 2216 activated PDC by inhibiting NGF induced CD271 signaling. TAT-Pep5 by itself and/or DMSO (solvent for TAT-Pep5) did not alter IFNα secretion in ODN 2216 activated PDC compared to ODN 2216 activated PDC without NGF (Fig. 6b).

### Example 7: NGF promotes antigen mediated CD4⁺ T-cell proliferation

PDCs are professional antigen presenting cells. PDCs were purified using BDCA4⁺ microbeads and CD4⁺ T-cells by negative selection from peripheral blood of patients with allergy against certain known allergens such as grass antigen and guinea pig antigen. Purified and CFSE (carboxyflourescein succinimidyl ester) labeled CD4⁺ T-cells were co-cultured in duplicates with autologous PDCs in the presence of optimal concentration of allergy specific allergen/antigen with and without NGF at 5, 25 and 100 ng/ml. After 5 days in culture the proliferation of CD4⁺ T-cells was determined by diminishing CFSE fluorescence. As shown in Fig. 7a, NGF at 25 ng/ml demonstrated significantly increased antigens (allergen) mediated CD4⁺ T-cells proliferation compared with autologous CD4⁺ T cell/PDC co-cultured without NGF. However, with NGF 100 ng/ml T-cells proliferation was considerably reduced. In contrast, with control antigen very little proliferation was noticed. T-cells on its own (without PDC co-culture) did not show any proliferation whether incubated with or without antigen (allergen) plus minus NGF or with NGF alone.

### Example 8: NGF reduces secretion of Th1 cytokines IL2 and IFNγ by T-cells

Murine PDCs were generated from bone marrow cells. Bone marrow cells were isolated by flushing murine femur and tibia. Erythrocytes were lysed using ACK lysis buffer (Life Technologies). Remaining cells were washed and cultured at a density of 2×106cells/ml in RPMI 1640 medium (Life Technologies) supplemented with 10% FCS, 1mM sodium pyruvat, 2mM L-glutamine, 100 IU/ml penicillin, 100µg/ml streptomycin, 10mM HEPES buffer and 0.1mM β-mercaptoethanol. To differentiate bone marrow cells into pDCs, 100ng/ml fms-like tyrosine kinase 3-ligand (Flt3-L) was added to the cells. After 8 days of culture pDCs were enriched by removing the CD11b+ cells from the non-adherent cell fraction using CD11b MicroBeads (Miltenyi Biotec) according to manufacturer instructions. Dead cells were excluded using Dead cell removal Kit (Miltenyi Biotec). Purity was greater than 95% as evaluated by flow cytometry using following antibodies: CD11c-APC Cy7 (BD Biosciences), B220-PerCP (Miltenyi Biotec), SiglecH-PE (eBioscience). Murine T-cells were isolated from the OTII mouse strain expressing mouse alpha-chain and beta-chain T cell receptor specific for chicken ovalbumin 323-339, using the CD+ T-cell isolation kit (Miltenyi Biotec).

Both, pDCs and T-cells were cultured overnight with or without the chicken ovalbumin peptide 323-339 in the presence or absence of NGF (100ng/ml). The concentrations of T-cell secreted Th1-cytokines IL-2 and IFNγ in the supernatant were determined by using the CBA kit (BD Bioscience) following manufacturer's instruction with modification in data analysis using Microsoft Excel.

Figure 5 shows the influence of NGF on the secretion of the Th1 cytokines IFNγ and IL-2 in the co-culture. In the presence of p75^{NTR+/+} PDCs presenting the ovalbumin peptide (OVA) to the T-cells, T-cells secrete the Th1 cytokines IFNγ (Fig.5a) and IL-2 (Fig. 5b). Compared to co-culture with p75^{NTR-/-} PDCs, T-cells co-cultured with PDCs from the p75^{NTR+/+} strain react with reduced secretion of both Th1 cytokines upon addition of NGF.

### Example 9: p75NTR knock out inhibits Th2 immune responses and blocks tolerance development

Heterozygous p75NTR mice were purchased from The Jackson Laboratory (Bar Harbor, Maine, USA) and bred under pathogen-free conditions in the animal facility of the TU Dresden. Male and female p75NTR+/+ and p75NTR-/- mice were used at 10-12 weeks of age for experiments.

### Generation of PDCs

Plasmacytoid dendritic cells (PDCs) were generated *in vitro* as follows: Bone marrow cells were isolated by flushing femur and tibia of mice. Erythrocytes were lysed using ACK buffer. Remaining cells were washed and cultured at a density of 2×10⁶ cells per ml in RPMI 1640 medium supplemented with 10% FCS, 1mM sodium pyruvat, 2mM L-glutamine, 100 IU per ml penicillin, 100µg/ml streptomycin, 10mM HEPES buffer and 0.1mM β-mercaptoethanol. To differentiate bone marrow cells into PDCs, 100ng/ml Flt3-L (fms-like tyrosine kinase 3-ligand) was added to the cells. After 8 days of culture PDCs were enriched by removing the CD11b⁺ fraction using CD11b Microbeads (Miltenyi Biotec) according to manufacturer instructions. Dead cells were excluded using Dead cell removal Kit (Miltenyi Biotec). Purity of PDCs was evaluated by flow cytometry (CD11b⁻CD11c⁺B220⁺Siglec-H⁺).

### Allergic asthma induction in mice

To induce allergic asthma, *in vitro* generated PDCs were incubated with 100µg/ml ovalbumin (OVA; grade V; Sigma-Aldrich) for 24h. To sensitize mice, 1×10⁶ OVA-loaded PDCs were injected intratracheally to the lungs of anesthetized mice using 24GA i.v. cannula (BD Neoflon™). Control animals received either the same amount of PDCs without OVA or PBS. After 10 days, mice were exposed to 1% w/v OVA-aerosol for 30min on 3 consecutive days in order to induce allergic reaction. 24h after last provocation, animals were sacrificed and the immune reaction was examined based on the bronchoalveolar lavage fluid (BALF) cell composition (eosinophils, lymphocytes, macrophages) and pro-inflammatory cytokines spectrum, lung histology and blood serum OVA-specific IgE levels.

### BALF cells analysis

BALF cells were quantified by flow cytometry. Cells were preincubated with FcR blocking reagent to avoid a non-specific binding. The following antibodies were used for staining: CD3-V500, CD4-V450, CD8-PE Cy7, CD11c-APC Cy7, B220-PE, F4/80-PerCP, SiglecF-AF647, Ly6G-FITC. Among lymphocytes (FSC^{low}/ SSC^{low}) the CD4⁺ T helper cells were designated as CD3^{pos}CD4^{pos}; the CD8⁺ T helper cells as CD3^{pos}CD8^{pos}; B-cells as CD3^{neg}B220^{pos}; among granulocytes (FSC^{low}/ SSC^{high}, Ly6G^{pos}) eosinophils were assigned as SiglecF^{pos}CD11c^{neg} and neutrophiles as SiglecF^{neg}. Macrophages were assigned as FSC^{high} highly autofluorescent CD11c^{pos}F4/80^{pos} cells. Additionally, the cytospins of collected cells were prepared and stained according to Pappenheim's method.

### Quantification of BALF cytokines

BALF supernatant was used for quantification of IL-4, IL-5 and IL-13 by ELISA (eBioscience) according to manufacturer instruction.

### Histological analysis

Lungs were perfused with PBS and fixed in 4% v/v formaldehyde. 4µm sections of paraffin-embedded lungs were stained with PAS staining for quantification of inflammation and Goblet cell hyperplasia.

To investigate the role of p75NTR expressed on PDCs in the process of disease triggering, we used the mouse model of OVA-mediated allergic asthma. OVA-pulsed PDCs from mice were intratracheally applied to the lung of either p75NTR+/+ or p75NTR-/- mice. After provocation with OVA aerosol characteristic symptoms of asthma like severe eosinophilia, lung inflammation and intensive mucus production were analyzed. p75NTR+/+ mice treated with OVA-loaded p75NTR-/- PDCs showed significantly reduced numbers of immune cells in the BALF (lymphocytes and eosinophils) compared to mice that received p75NTR+/+ PDCs (Fig. 2a, b). OVA-mediated immune response further lead to increased Th2 cytokine secretion (IL-4, IL-5 and IL-13) in the BALF of mice treated with p75NTR+/+ PDCs but not in mice that received p75NTR-/- PDCs (Fig. 2c). Perivascular inflammation and Goblet cell hyperplasia in the lung were diminished in mice treated with p75NTR-/- PDCs compared to mice treated with p75NTR+/+ PDCs (Fig. 2d, e). In summary, mice that received PDCs lacking p75NTR developed significantly less allergic asthma.

It has known in the art that blocking or deleting of p75NTR in mice prevented the development of lung inflammation and airway hyperresponsiveness. In the present study, however, it could be shown for the first time that allergic asthma can be induced in p75NTR-/- mice by intratracheal application of p75NTR+/+ PDCs loaded with OVA. In contrast, application of OVA-loaded p75NTR-/- PDCs did not induce asthma. In detail, immune cells are significantly increased in the BALF of p75NTR-/- mice that received p75NTR+/+ PDCs (Fig. 2a, b). In addition, Th2 cytokine profile (IL-4, IL-5 and IL-13) is significantly enhanced compared to mice that received p75NTR-/- PDCs (Fig. 2c). Histological examination of lung tissue revealed that mice treated with p75NTR+/+ PDCs developed severe perivascular inflammation and Goblet cell hyperplasia compared to mice that received p75NTR-/- PDCs (Fig. 2d, e).

## Claims

1. A screening method for agonists and antagonists of p75^{NTR} signaling comprising the steps of:
• Contacting primary or *in vitro* generated human or animal plasmacytoid dendritic cells (PDC), or PDC cell lines that expresses the nerve growth factor receptor p75^{NTR} with a test substance;
• Incubating said contacted human or animal PDCs or PDC cell lines for a period of time, which is sufficient for effecting p75^{NTR} signaling;
• Determining the effect of the test substance on the primary or *in vitro* generated human or animal PDCs or PDC cell lines;
• Comparing the effect of the test substance in the contacted primary or *in vitro* generated human or animal PDCs or PDC cell lines with control cells or cell lines; and
• Selecting a test substance that agonizes or antagonizes p75^{NTR} signaling in primary or *in vitro* generated human or animal PDCs or PDCs cell lines.

2. The screening method of claim 1, wherein the human or animal PDCs or PDC cell lines express the nerve growth factor receptor p75^{NTR} and/or at least one protein selected from the group of Toll like receptors, preferably TLR4, TLR7 or TLR9.

3. The screening method of claim 1 or 2, wherein the human or animal PDCs or PDC cell lines are have been genetically modified to overexpress p75^{NTR} and/or at least one protein selected from the group consisting of TLR9, TLR7, TRAF3 and TRAF6.

4. The screening method according to any one of the preceding claims wherein the control cells or cell lines are primary cells, most suitably PDCs; or cells which do not naturally express p75^{NTR} or cells in which p75^{NTR} is knocked out or cells in which the expression of p75^{NTR} is blocked, reduced or inhibited.

5. The method according to any one of the preceding claims, wherein the PDCs or PDC cell lines that expresses p75^{NTR} are co-incubated with T-cells, comprising the steps of:
• Contacting a human or animal PDCs or PDC cell line that express the nerve growth factor receptor p75NTR, which are co-incubated with T-cells, with a test substance ;
• Incubating said contacted co-culture of said human or animal PDCs or PDC cell line and said T-cells for a period of time, which is sufficient for effecting p75^{NTR} signaling;
• Determining the effect of the test substance on the PDCs or PDC cell lines and/or on the T-cells;
• Comparing of the effect of the test substance in the contacted PDCs or PDC cell lines and/or T-cells with control cells or cell lines and/or T-cells; and
• Selecting a test substance that agonizes or antagonizes p75^{NTR} signaling.

6. The screening method according to any one of the preceding claims, wherein the step of contacting a human or animal PDCs or PDC cell lines that express the nerve growth factor receptor p75^{NTR}, with said test substance, is performed in the presence of a natural ligand of p75^{NTR} under conditions allowing the interaction of the test substance and the p75^{NTR} protein and/or the interaction of the test substance with the natural ligand of p75^{NTR}.

7. The screening method according to any one of the preceding claims, wherein the PDCs or PDC cell lines are pre-activated prior to or during their use in the screening method, suitably with at least one agonist of Toll like receptor signaling.

8. The screening method according to any one of the preceding claims, wherein the antagonistic or agonistic effect of the test substance on the p75^{NTR} signaling in the assay is measured based on the expression analysis of cytokines or the analysis of intracellular signaling cascades or the surface marker expression analysis or the measurement of the uptake, intracellular processing and presentation of external antigens or the analysis of T-cell proliferation.

9. The screening method according to any one of the preceding claims, wherein said method is performed *in vivo,* **characterized in that** the PDCs or PDC cell lines which express p75^{NTR} and/or at least one Toll like receptor, preferably TLR7 and/or TLR9, were administered to an animal model, e.g. an animal model, which is specific for an immune, inflammatory or proliferative disease such as an OVA induced allergic asthma model, another model of allergic diseases, EAE model, diabetes model, SLE model, transplantation model, GVHD model and tumor model.

10. The screening method of claim 9, wherein the determination of the antagonistic or agonistic effect of a test substance in said animal models are performed in the presence of control animals, preferably control animals which comprise at least the PDCs but in which p75^{NTR} is not expressed or expressed at lower levels, or wherein the applied PDCs or PDCs cell lines exhibit reduced, blocked or inhibited expression of p75^{NTR}.

11. A combination comprising at least one modulator of p75^{NTR} signalling in PDCs selected from a p75^{NTR} antagonist or p75^{NTR} agonist and at least one agonist of TLR9, wherein said agonist of p75^{NTR} signalling is selected from
i) the group comprising NGF, BDNF, NT-3, NT-4, NT-5;
ii) an activating antibody, which is anti-p75^{NTR} antibody MC192;
iii) activating peptides and activating small molecules selected from, LM11A-24 or LM11A-31 and LM11A-36.
wherein said antagonist of p75^{NTR} signalling is selected from
i) the group comprising pro-NGF, pro-BDNF, pro-NT-3, pro-NT-4, and pro-NT-5;
ii) blocking antibodies selected from anti human p75^{NTR} monoclonal antibody clones ME20.4, MLR2, HB-8737, NGFR5 and humanized versions thereof;AB1554;
iii) ; and Tanezumab, PG110, REGN475, and Fulranumab;
iv) the blocking peptide tat-PEP5; v) peptides that block the interaction of p75^{NTR} with TRAF6 which are peptides binding to the protein motif EGEKLHSDSGISVDS (SEQ ID No. 1) from the intracellular domain of p75^{NTR}; and TRAF6 decoy peptides comprising the RPTIPRNPK peptide (SEQ ID No. 2);
v) small molecules selected from derivatives of 2-oxo-alkyl-1-piperazin-2-one, PD 90780, ALE-0540, Ro 08-2750 and Y1036;
vi) morpholinos that block expression of p75^{NTR}; or
vii) an shRNA or RNAi that blocks expression of p75^{NTR} or downstream signalling; and
wherein said agonist of TLR9 is selected from bacterial DNA, CPG-ODNs Class A, which are selected from ODN 1585, ODN 2216, ODN 2336; CPG-ODNs Class B, which are selected from ODN BW006, ODN D-SL01, ODN 1668; ODN 1826, ODN 2006, ODN 2007, and CPG-ODNs Class C, which are selected from ODN D-SL03, ODN 2395, ODN M362 and ODN C792.

12. A pharmaceutical composition comprising the combination claim 11.

13. The pharmaceutical composition of claim 12, which is a vaccine composition.

14. The vaccine composition according to claim 13, further comprising at least one immune stimulating agent, which is selected from monophosphoryl lipid A, muramyl dipeptide, oligodeoxynucleotides, double-stranded RNA, alternative pathogen-associated molecular patterns, saponins, resiquimod, cytokines, chemokines and antigens from *Mycobacterium tuberculosis.*

15. The vaccine composition according to claim 13 or 14, further comprising at least one agent selected from insoluble aluminium compounds, calcium phosphate, liposomes, microparticles, emulsions, virus-like particles and viral vectors.

16. The vaccine composition according to 13, further comprising isolated p75^{NTR} expressing PDCs, *in vitro* generated p75^{NTR} expressing PDCs, or a p75^{NTR} expressing PDC cell line.

17. The combination according to claim 11 for use as vaccine adjuvant.

18. The combination according to claim 11 or the pharmaceutical or vaccine composition according any one of claims 12-17 for use in the prevention and/or treatment of a disease selected from the group consisting of central and peripheral neurodegenerative diseases, senile dementia, epilepsy, Alzheimer's disease, Parkinson's disease, Huntington's disease, Down's syndrome, prion diseases, amnesia, schizophrenia, depression, bipolar disorder, amyotrophic lateral sclerosis, multiple sclerosis, cardiovascular conditions, post-ischemic cardiac damage, cardiomyopathies, myocardial infarction, heart failure, cardiac ischemia, cerebral infarction, peripheral neuropathies, damage to the optic nerve and/or to the retina, retinal pigment degeneration, glaucoma, retinal ischemia, macular degeneration, spinal cord traumas, cranial traumas, atherosclerosis, stenosis, wound healing disorders, alopecia, any type of cancer, any type of tumors, any type of metastases, any type of leukemia, respiratory disorders, pulmonary inflammation, allergy, anaphylaxis, asthma, atopic dermatitis, chronic obstructive pulmonary disease, cutaneous pain, somatic pain, visceral pain, neurological pain, chronic neuropathic pain, inflammatory pain, autoimmune diseases, rheumatoid arthritis (polyarthritis, oligoarthritis), ankylosing spondylitis, collagenosis, systemic lupus erythematodes (SLE), SHARP syndrome, Sjögren's syndrome, scleroderma, polymyositis, dermatomyositis, progressive systemic sclerosis, spondyloarthritis (Morbus Bechterew, reactive arthritis, enteropathic arthritis, psoriatic arthritis, undifferentiated spondyloarthritis), rheumatic fever, Aicardi-Goutières syndrome, vasculitis, Wegener's granulomatosis disease, nephritis, stroke, ulcerative colitis, Crohn's diesease, Morbus Whipple, scleroderma, Still's disease, bronchopulmonary dysplasia (BPD), bronchiolitis, RSV-associated bronchiolitis, Diabetes mellitus, fibromyalgia syndrome, coeliac disease, Hashimoto's disease, hypothyroidism, hyperthyroidism, Addison's disease, graft versus host disease (GVHD), autoimmune thrombocytopenia, autoimmune hemolytic anemia, Löfgren syndrome, Behcet disease, nephrotic syndrome, uveitis, psoriatic arthritis, psoriasis (plaque psoriasis, pustular psoriasis), bone fractures, bone diseases, osteoporosis and all bacterial, fungal, viral infectious diseases, as well infections with eukaryotic parasites.

## Patentansprüche

1. Screening-Verfahren für Agonisten und Antagonisten der p75^{NTR}-Signalisierung, umfassend die Schritte:
• Kontaktieren von primären oder *in vitro* generierten humanen oder tierischen plasmazytoiden dendritischen Zellen (PDC) oder PDC-Zelllinien, die den Rezeptor des Nervenwachstumsfaktors p75^{NTR} exprimieren, mit einer Testsubstanz;
• Inkubieren der kontaktierten menschlichen oder tierischen PDCs oder PDC-Zelllinien für eine Zeitspanne, die ausreichend ist, um die p75^{NTR}-Signalisierung zu bewirken;
• Bestimmung der Wirkung der Testsubstanz auf die primären oder *in vitro* erzeugten humanen oder tierischen PDCs oder PDC-Zelllinien;
• Vergleich der Wirkung der Testsubstanz in den kontaktierten primären oder *in vitro* erzeugten humanen oder tierischen PDCs oder PDC-Zelllinien mit Kontrollzellen oder Zelllinien; und
• Auswahl einer Testsubstanz, die die p75NTR-Signalisierung in primären oder *in vitro* erzeugten humanen oder tierischen PDCs oder PDCs Zelllinien aktiviert oder hemmt.

2. Screeningverfahren nach Anspruch 1, wobei die humanen oder tierischen PDCs oder PDC-Zellinien den Rezeptor des Nervenwachstumsfaktors p75^{NTR} und/oder mindestens ein Protein ausgewählt aus der Gruppe Toll-artiger Rezeptoren, vorzugsweise TLR4, TLR7 oder TLR9, exprimieren.

3. Screeningverfahren nach Anspruch 1 oder 2, wobei die humanen oder tierischen PDCs oder PDC-Zellinien genetisch modifiziert wurden, um p75^{NTR} und/oder mindestens ein Protein, ausgewählt aus der Gruppe bestehend aus TLR9, TLR7, TRAF3 und TRAF6, überzuexprimieren.

4. Screening-Verfahren nach einem der vorhergehenden Ansprüche, wobei die Kontrollzellen oder Zelllinien primäre Zellen sind, am geeignetsten PDCs; oder Zellen, die p75^{NTR} nicht natürlich exprimieren oder Zellen, in denen p75^{NTR} abgeschaltet ist oder Zellen, in denen die Expression von p75NTR blockiert, reduziert oder inhibiert ist.

5. Verfahren nach einem der vorhergehenden Ansprüche, wobei die PDCs oder PDC-Zelllinien, die p75^{NTR} exprimieren, mit T-Zellen coinkubiert werden, umfassend die Schritte:
• Kontaktieren menschlicher oder tierischer PDCs oder PDC-Zelllinie, die den Nervenwachstumsfaktor-Rezeptor p75^{NTR} exprimieren, die mit T-Zellen co-inkubiert werden, mit einer Testsubstanz;
• Inkubieren der kontaktierten Co-Kultur der menschlichen oder tierischen PDCs oder PDC-Zelllinie und der T-Zellen für eine Zeitspanne, die ausreichend ist, um die p75^{NTR}-Signalisierung zu bewirken;
• Bestimmung der Wirkung der Testsubstanz auf die PDCs oder PDC-Zelllinien und / oder auf die T-Zellen;
• Vergleich der Wirkung der Testsubstanz in den kontaktierten PDCs oder PDC-Zelllinien und/oder T-Zellen mit Kontrollzellen oder Zellinien und/oder T-Zellen; und
• Auswahl einer Testsubstanz, die die p75^{NTR}-Signalisierung aktiviert oder hemmt.

6. Screening-Verfahren nach einem der vorhergehenden Ansprüche, wobei der Schritt des Kontaktierens von menschlichen oder tierischen PDCs oder PDC-Zelllinien, die den Rezeptor des Nervenwachstumsfaktors p75^{NTR} exprimieren, mit der Testsubstanz in Gegenwart eines natürlichen Liganden von p75^{NTR} unter Bedingungen durchgeführt wird, die die Wechselwirkung der Testsubstanz und des p75NTR-Proteins und/oder die Wechselwirkung der Testsubstanz mit dem natürlichen Liganden von p75^{NTR} ermöglichen.

7. Screening-Verfahren nach einem der vorhergehenden Ansprüche, wobei die PDCs oder PDC-Zelllinien vor oder während ihrer Verwendung in dem Screening-Verfahren voraktiviert werden, geeigneterweise mit mindestens einem Agonisten der Toll-ähnlichen-Rezeptor-Signalisierung.

8. Screening-Verfahren nach einem der vorhergehenden Ansprüche, wobei der antagonistische oder agonistische Effekt der Testsubstanz auf die p75^{NTR}-Signalisierung im Assay gemessen wird, basierend auf der Expressionsanalyse von Zytokinen oder der Analyse von intrazellulären Signalkaskaden oder der Oberflächenmarker-Expressions-Analyse oder der Messung der Aufnahme, intrazellulären Verarbeitung und Präsentation von externen Antigenen oder der Analyse der T-Zell-Proliferation.

9. Screening-Verfahren nach einem der vorhergehenden Ansprüche, wobei das Verfahren *in vivo* durchgeführt wird, **dadurch gekennzeichnet, dass** die PDCs oder PDC-Zelllinien, die p75^{NTR} und/oder mindestens einen Toll-like-Rezeptor, vorzugsweise TLR7 und/oder TLR9, exprimieren, einem Tiermodell, z. B. einem Tiermodell, das spezifisch für eine Immun-, Entzündungs- oder proliferative Erkrankung ist, wie einem OVA-induzierten allergischen Asthmamodell, einem anderen Modell von allergischen Erkrankungen, EAE-Modell, Diabetesmodell, SLE-Modell, Transplantationsmodell, GVHD-Modell und Tumormodell verabreicht wurden.

10. Screening-Verfahren nach Anspruch 9, wobei die Bestimmung der antagonistischen oder agonistischen Wirkung einer Testsubstanz in den Tiermodellen in Gegenwart von Kontrolltieren, vorzugsweise Kontrolltieren, die mindestens die PDCs umfassen, in denen aber p75^{NTR} nicht oder auf geringerem Niveau exprimiert wird, durchgeführt wird, oder wobei die angewendeten PDCs oder PDCs-Zelllinien eine reduzierte, blockierte oder inhibierte Expression von p75^{NTR} zeigen.

11. Kombination, umfassend mindestens einen Modulator der p75^{NTR}-Signalisierung in PDCs, ausgewählt aus einem p75^{NTR}-Antagonisten oder p75^{NTR}-Agonisten und mindestens einem Agonisten von TLR9, wobei der Agonist der p75^{NTR}-Signalisierung ausgewählt ist aus
i) der Gruppe umfassend NGF, BDNF, NT-3, NT-4, NT-5;
ii) einen aktivierenden Antikörper, der der anti-p75^{NTR}-Antikörper MC192 ist;
iii) aktivierenden Peptiden und aktivierenden kleinen Molekülen, ausgewählt aus LM11A-24 oder LM11A-31 und LM11A-36.
wobei der Antagonist der p75^{NTR}-Signalisierung ausgewählt ist aus
i) der Gruppe, umfassend Pro-NGF, Pro-BDNF, Pro-NT-3, Pro-NT-4 und Pro-NT-5;
ii) blockierenden Antikörpern ausgewählt aus den monoklonalen anti-humanen monoklonalen p75^{NTR}-Antikörperklonen ME20.4, MLR2, HB-8737, NGFR5 und humanisierten Versionen davon; AB1554;
iii) und Tanezumab, PG110, REGN475 und Fulranumab;
iv) dem blockierenden Peptid tat-PEP5; v) Peptiden, die die Wechselwirkung von p75NTR mit TRAF6 blockieren, die Peptide sind, die an das Proteinmotiv EGEKLHSDSGISVDS (SEQ ID Nr. 1) der intrazellulären Domäne von p75^{NTR} binden; und TRAF6-Decoy-Peptiden, umfassend das RPTIPRNPK-Peptid (SEQ ID Nr. 2);
v) kleinen Molekülen, ausgewählt aus Derivaten von 2-Oxo-alkyl-1-piperazin-2-on, PD 90780, ALE-0540, Ro 08-2750 und Y1036;
vi) Morpholinen, die die Expression von p75^{NTR} blockieren; oder
vii) einer shRNA oder RNAi, die die Expression von p75^{NTR} oder Downstream-Signalisierung blockiert; und
wobei der Agonist von TLR9 ausgewählt ist aus bakterieller DNA, CPG-ODNs Klasse A, die ausgewählt sind aus ODN 1585, ODN 2216, ODN 2336; CPG-ODNs Klasse B, die ausgewählt sind aus ODN BW006, ODN D-SL01, ODN 1668; ODN 1826, ODN 2006, ODN 2007 und CPG-ODNs Klasse C, die aus ODN D-SL03, ODN 2395, ODN M362 und ODN C792 ausgewählt sind.

12. Pharmazeutische Zusammensetzung, enthaltend die Kombination nach Anspruch 11.

13. Pharmazeutische Zusammensetzung nach Anspruch 12, welche eine Impfstoffzusammensetzung ist.

14. Impfstoffzusammensetzung nach Anspruch 13, weiterhin umfassend mindestens einen immunstimulierenden Wirkstoff, ausgewählt aus Monophosphoryllipid A, Muramyldipeptid, Oligodesoxynukleotiden, doppelsträngiger RNA, alternativen pathogenassoziierten molekularen Mustern, Saponinen, Resiquimod, Zytokinen, Chemokinen und Antigenen von *Mycobacterium tuberculosis.*

15. Impfstoffzusammensetzung nach Anspruch 13 oder 14, ferner umfassend mindestens ein Mittel, ausgewählt aus unlöslichen Aluminiumverbindungen, Kalziumphosphat, Liposomen, Mikropartikeln, Emulsionen, virusartigen Partikeln und viralen Vektoren.

16. Impfstoffzusammensetzung gemäß Anspruch 13, weiterhin umfassend isolierte p75^{NTR}-exprimierende PDCs, *in vitro* erzeugte p75^{NTR}-exprimierende PDCs oder eine p75^{NTR}-exprimierende PDC-Zelllinie.

17. Kombination nach Anspruch 11 zur Verwendung als Impfstoffadjuvans.

18. Die Kombination nach Anspruch 11 oder die pharmazeutische Zusammensetzung oder Impfstoffzusammensetzung nach einem der Ansprüche 12-17 zur Verwendung bei der Prävention und/oder Behandlung einer Krankheit ausgewählt aus der Gruppe bestehend aus zentralen und peripheren neurodegenerativen Erkrankungen, Altersdemenz, Epilepsie, Alzheimer-Krankheit, Parkinson-Krankheit, Huntington-Krankheit, Down-Syndrom, Prionenkrankheiten, Amnesie, Schizophrenie, Depression, bipolarer Störung, amyotropher Lateralsklerose, Multipler Sklerose, kardiovaskulären Zuständen, post-ischämischen Herzschäden, Kardiomyopathien, Myokardinfarkt, Herzinsuffizienz, Herz-Ischämie, Hirninfarkt, peripheren Neuropathien, Schädigung des Sehnervs und/oder der Netzhaut, retinaler Pigmentdegeneration, Glaukom, retinaler Ischämie, Makuladegeneration, Rückenmarkstraumata, Schädeltraumata, Atherosklerose, Stenose, Wundheilungsstörungen, Alopezie, beliebigen Arten von Krebs, jeder Art von Tumoren, jeder Art von Metastasen, jeder Art von Leukämie, respiratorischen Erkrankungen, Lungenentzündung, Allergie, Anaphylaxie, Asthma, atopischer Dermatitis, chronisch obstruktiver Lungenerkrankung, Hautschmerz, somatischem Schmerz, visceralem Schmerz, neurologischem Schmerz, chronischem neuropathischen Schmerz, entzündlichem Schmerz, Autoimmunerkrankungen, rheumatoider Arthritis (Polyarthritis, Oligoarthritis), Spondylitis ankylosans, Kollagenose, systemischem Lupus erythematodes (SLE), SHARP-Syndrom, Sjögren-Syndrom, Sklerodermie, Polymyositis, Dermatomyositis, progressiver systemischer Sklerose, Spondylarthritis (Morbus Bechterew, reaktive Arthritis, Enteropathische Arthritis, Psoriasis-Arthritis, undifferenzierte Spondyloarthritis), rheumatischem Fieber Aikardi-Goutières-Syndrom, Vaskulitis, Wegener-Granulomatose, Nephritis, Schlaganfall, Colitis ulcerosa, Morbus Crohn, Morbus Whipple, Sklerodermie, Morbus Still, Bronchopulmonaler Dysplasie (BPD), Bronchiolitis, RSV-assoziierter Bronchiolitis, Diabetes mellitus, Fibromyalgie-Syndrom, Zöliakie, Hashimoto-Krankheit, Hypothyreoidismus, Hyperthyreose, Addison-Krankheit, Graft-versus-Host-Krankheit (GVHD), autoimmuner Thrombozytopenie, autoimmuner hämolytischer Anämie, Löfgren-Syndrom, Behcet-Krankheit, nephrotischem Syndrom, Uveitis, Psoriasis-Arthritis, Psoriasis (Plaque-Psoriasis, Psoriasis pustulosa), Knochenbrüchen, Knochenerkrankungen, Osteoporose und allen bakteriellen, fungalen, viralen Infektionskrankheiten sowie Infektionen mit eukaryotischen Parasiten.

## Revendications

1. Méthode de recherche par criblage d'agonistes et d'antagonistes de la signalisation de p75^{NTR}, comprenant les étapes consistant à :
• Mettre en contact des lignées cellulaires PDC ou des cellules dendritiques plasmacytoïdes (PDC) humaines ou animales primaires ou générées *in vitro* exprimant le récepteur du facteur de croissance nerveuse p75^{NTR} avec une substance d'essai ;
• Incuber lesdites lignées cellulaires PDC ou lesdites cellules PDC humaines ou animales pendant une période suffisante pour mettre en oeuvre la signalisation de p75^{NTR} ;
• Déterminer l'effet de la substance d'essai sur les lignées cellulaires PDC ou les PDC humaines ou animales primaires ou générées *in vitro ;*
• Comparer l'effet de la substance d'essai dans les lignées cellulaires PDC ou les PDC humaines ou animales primaires ou générées *in vitro* mises en contact à celui dans les cellules ou lignées cellulaires témoins ; et
• Sélectionner une substance d'essai agoniste ou antagoniste de la signalisation de p75^{NTR} dans les lignées cellulaires PDC ou les PDC humaines ou animales primaires ou générées *in vitro.*

2. Méthode de recherche par criblage selon la revendication 1, où les lignées cellulaires PDC ou les PDC humaines ou animales expriment le récepteur de facteur de croissance nerveuse p75^{NTR} et/ou au moins une protéine choisie dans le groupe constitué par les récepteurs TLR, préférentiellement TLR4, TLR7 ou TLR9.

3. Méthode de recherche par criblage selon la revendication 1 ou 2, où les lignées cellulaires PDC ou les PDC humaines ou animales ont été génétiquement modifiées pour surexprimer p75^{NTR} et/ou au moins une protéine choisie dans le groupe constitué par TLR9, TLR7, TRAF3 et TRAF6.

4. Méthode de recherche par criblage selon l'une quelconque des revendications précédentes, où les lignées cellulaires ou cellules témoins sont des cellules primaires, de façon plus adaptée des PDC ; ou des cellules qui n'expriment pas naturellement p75^{NTR}, ou encore des cellules dans lesquelles p75^{NTR} a été invalidé, ou encore des cellules dans lesquelles l'expression de p75^{NTR} est bloquée, réduite ou inhibée.

5. Méthode selon l'une quelconque des revendications précédentes, où les lignées cellulaires PDC ou les PDC exprimant p75^{NTR} sont co-incubées avec des cellules T, comprenant les étapes consistant à :
• Mettre en contact une lignée cellulaire PDC ou des PDC humaines ou animales exprimant le récepteur de facteur de croissance nerveuse p75^{NTR}, qui sont co-incubées avec des cellules T, avec une substance d'essai ;
• Incuber ladite co-culture mise en contact desdites lignées cellulaires PDC ou desdites cellules PDC humaines ou animales avec lesdites cellules T pendant une période suffisante pour mettre en oeuvre la signalisation de p75^{NTR} ;
• Déterminer l'effet de la substance d'essai sur les lignées cellulaires PDC ou les PDC et/ou sur les cellules T ;
• Comparer l'effet de la substance d'essai dans les lignées cellulaires PDC ou les PDC mises en contact et/ou les cellules T avec celui dans des lignées cellulaires ou des cellules témoin et/ou des cellules T ; et
• Sélectionner une substance d'essai agoniste ou antagoniste de la signalisation de p75^{NTR}.

6. Méthode de recherche par criblage selon l'une quelconque des revendications précédentes, où l'étape de mise en contact de lignées cellulaires PDC ou de PDC humaines ou animales exprimant le récepteur de facteur de croissance nerveuse p75^{NTR} avec ladite substance d'essai est mise en oeuvre en présence d'un ligand naturel de p75^{NTR} dans des conditions permettant l'interaction de la substance d'essai et de la protéine p75^{NTR} et/ou l'interaction de la substance d'essai avec le ligand naturel de p75^{NTR}.

7. Méthode de recherche par criblage selon l'une quelconque des revendications précédentes, où les lignées cellulaires PDC ou les PDC sont préactivées avant ou pendant leur utilisation dans la méthode de recherche par criblage, de façon adaptée avec au moins un agoniste de la signalisation de TLR.

8. Méthode de recherche par criblage selon l'une quelconque des revendications précédentes, où l'effet antagoniste ou agoniste de la substance d'essai sur la signalisation de p75^{NTR} dans le dosage est mesuré en fonction de l'analyse de l'expression des cytokines ou l'analyse des cascades de signalisation intracellulaire, ou encore l'analyse de l'expression des marqueurs de surface, ou encore la mesure de la capture, de la transformation intracellulaire et de la présentation des antigènes externes, ou encore l'analyse de la prolifération des cellules T.

9. Méthode de criblage selon l'une quelconque des revendications précédentes, où ladite méthode est mise en oeuvre *in vivo,* **caractérisée en ce que** les lignées cellulaires PDC ou les PDC qui expriment p75^{NTR} et/ou au moins un récepteur TLR, préférentiellement TLR7 et/ou TLR9, ont été administrées à un modèle animal, par exemple un modèle animal qui est spécifique d'une maladie immune, inflammatoire ou proliférative, telle qu'un modèle d'asthme allergique induit par OVA, un autre modèle de maladies allergiques, un modèle d'EAE, un modèle de diabète, un modèle de LED, un modèle de transplantation, un modèle de maladie greffon contre hôte et un modèle de tumeur.

10. Méthode de recherche par criblage selon la revendication 9, où la détermination de l'effet antagoniste ou agoniste d'une substance d'essai dans lesdits modèles animaux est mise en oeuvre en présence d'animaux témoins, préférentiellement d'animaux témoins qui comprennent au moins les PDC mais dans lesquels p75^{NTR} n'est pas exprimé ou exprimé à des niveaux inférieurs, ou où les lignées cellulaires PDC ou les PDC appliquées présentent une expression réduite, bloquée ou inhibée de p75^{NTR}.

11. Combinaison comprenant au moins un modulateur de la signalisation de p75^{NTR} dans les PDC choisi parmi un antagoniste de p75^{NTR} et un agoniste de p75^{NTR} et au moins un agoniste de TLR9, où ledit agoniste de la signalisation de p75^{NTR} est choisi parmi
i) le groupe comprenant NGF, BDNF, NT-3, NT-4, NT-5 ;
ii) un anticorps d'activation, qui est l'anticorps anti-p75^{NTR} MC192 ;
iii) des peptides d'activation et des petites molécules d'activation choisies parmi LM11A-24 ou LM11A-31 et LM11A-36.
où ledit antagoniste de signalisation de p75^{NTR} est choisi parmi
i) le groupe comprenant pro-NGF, pro-BDNF, pro-NT-3, pro-NT-4 et pro-NT-5 ;
ii) des anticorps bloquant choisis parmi les clones d'anticorps monoclonaux anti-p75^{NTR} humain ME20.4, MLR2, HB-8737, NGFR5 et leurs versions humanisées ; AB1554 ;
iii) et Tanézumab, PG110, REGN475 et Fulranumab ;
iv) le peptide bloquant tat-PEP5 ;
v) les peptides qui bloquent l'interaction de p75^{NTR} avec TRAF6, qui sont des peptides se liant au motif protéinique EGEKLHSDSGISVDS (SEQ ID No. 1) du domaine intracellulaire de p75^{NTR} ; et des peptides leurres TRAF6 comprenant le peptide RPTIPRNPK (SEQ ID No. 2) ;
v) les petites molécules choisies parmi les dérivés de 2-oxo-alkyl-1-pipérazin-2-one, PD 90780, ALE-0540, Ro 08-2750 et Y1036 ;
vi) les groupements morpholino bloquant l'expression de p75^{NTR} ; ou
vii) un shARN ou un ARNi qui bloque l'expression de p75^{NTR} ou de la signalisation en aval ; et
où ledit agoniste de TLR9 est choisi parmi l'ADN bactérien, les CPG-ODN de classe A, qui sont choisis parmi ODN 1585, ODN 2216, ODN 2336 ; les CPG-ODN de classe B, qui sont choisis parmi ODN BW006, ODN D-SL01, ODN 1668 ; ODN 1826, ODN 2006, ODN 2007, et les CPG-ODN de classe C, qui sont choisis parmi ODN D-SL03, ODN 2395, ODN M362 et ODN C792.

12. Composition pharmaceutique comprenant la combinaison selon la revendication 11.

13. Composition pharmaceutique selon la revendication 12, qui est une composition de vaccin.

14. Composition de vaccin selon la revendication 13, comprenant en outre au moins un agent immunostimulant, qui est choisi parmi monophosphoryl-lipide A, muramyl-dipeptide, oligodésoxynucléotides, ARN double brin, motifs moléculaires associés à des pathogènes alternatifs, saponines, résiquimod, cytokines, chimiokines et antigènes de *Mycobacterium tuberculosis.*

15. Composition de vaccin selon la revendication 13 ou 14, comprenant en outre au moins un agent choisi parmi les composés d'aluminium insolubles, le phosphate de calcium, les liposomes, les microparticules, les émulsions, les particules semblables aux virus et les vecteurs viraux.

16. Composition de vaccin selon la revendication 13, comprenant en outre des PDC isolées exprimant p75^{NTR}, des PDC générées *in vitro* exprimant p75^{NTR} ou une lignée cellulaire PDC exprimant p75^{NTR}.

17. Combinaison selon la revendication 11 pour utilisation comme adjuvant de vaccin.

18. Combinaison selon la revendication 11 ou composition pharmaceutique ou de vaccin selon l'une quelconque des revendications 12 à 17 pour utilisation dans le traitement prophylactique et/ou thérapeutique d'une maladie choisie dans le groupe constitué par les suivantes : maladies neurodégénératives centrales et périphériques, démence sénile, épilepsie, maladie d'Alzheimer, maladie de Parkinson, chorée de Huntington, syndrome de Down, maladies à prions, amnésie, schizophrénie, dépression, trouble bipolaire, sclérose latérale amyotrophique, sclérose en plaques, états pathologiques cardio-vasculaires, lésions cardiaques post-ischémiques, myocardiopathies, infarctus du myocarde, défaillance cardiaque, ischémie cardiaque, infarctus cérébral, neuropathies périphériques, lésions du nerf optique et/ou de la rétine, dégénérescence pigmentaire rétinienne, glaucome, ischémie rétinienne, dégénérescence maculaire, traumatismes de la moelle épinière, traumatismes crâniens, athérosclérose, sténose, troubles de guérison des blessures, alopécie, tous les types de cancer, tous les types de tumeurs, tous les types de métastases, tous les types de leucémie, troubles respiratoires, inflammation pulmonaire, allergies, anaphylaxie, asthme, dermatite atopique, bronchopneumopathie chronique obstructive, douleur cutanée, douleur somatique, douleur viscérale, douleur neurologique, douleur neuropathique chronique, douleur inflammatoire, maladies auto-immunes, polyarthrite rhumatoïde (polyarthrite, oligoarthrite), spondylite ankylosante, collagénose, lupus érythémateux disséminé (LED), syndrome de Sharp, syndrome de Sjögren, sclérodermie, polymyosite, dermatomyosite, sclérose systémique progressive, spondylarthrite (Morbus Bechterew, arthrite réactive, arthrite entéropathique, arthrite psoriasique, spondylarthrite indifférenciée), fièvre rhumatismale, syndrome d'Aicardi-Goutières, vascularite, granulomatose de Wegener, néphrite, accident vasculaire cérébral, rectocolite hémorragique, maladie de Crohn, maladie de Whipple, sclérodermie, maladie de Still, dysplasie broncho-pulmonaire, bronchiolite, bronchiolite associée au VRS, diabète sucré, syndrome de fibromyalgie, maladie coeliaque, thyroïdite de Hashimoto, hypothyroïdie, hyperthyroïdie, maladie d'Addison, maladie greffon contre hôte, thrombocytopénie auto-immune, anémie hémolytique auto-immune, syndrome de Löfgren, maladie de Behçet, syndrome néphrotique, uvéite, arthrite psoriasique, psoriasis (psoriasis en plaques, psoriasis pustuleux), fractures osseuses, maladies osseuses, ostéoporose et toutes les maladies infectieuses bactériennes, fongiques, virales, ainsi que les infections par des parasites eucaryotes.


### SEQUENCE LISTING

<110> Technische Universität Dresden
<120> Cell-based assay and screening methods for modulators of p75NTR signaling
<130> TUD106EP
<160> 4
<170> PatentIn version 3.5
<210> 1
   <211> 15
   <212> PRT
   <213> Homo sapiens
<400> 1
   Glu Gly Glu Lys Leu His Ser Asp Ser Gly Ile Ser Val Asp Ser
   1               5                   10                  15
<210> 2
   <211> 9
   <212> PRT
   <213> Homo sapiens
<400> 2
   Arg Pro Thr Ile Pro Arg Asn Pro Lys
   1               5
<210> 3
   <211> 427
   <212> PRT
   <213> Homo sapiens
<400> 3
   Met Gly Ala Gly Ala Thr Gly Arg Ala Met Asp Gly Pro Arg Leu Leu
   1               5                   10                  15
   Leu Leu Leu Leu Leu Gly Val Ser Leu Gly Gly Ala Lys Glu Ala Cys
               20                  25                  30
   Pro Thr Gly Leu Tyr Thr His Ser Gly Glu Cys Cys Lys Ala Cys Asn
           35                  40                  45
   Leu Gly Glu Gly Val Ala Gln Pro Cys Gly Ala Asn Gln Thr Val Cys
       50                  55                  60
   Glu Pro Cys Leu Asp Ser Val Thr Phe Ser Asp Val Val Ser Ala Thr
   65                  70                  75                  80
   Glu Pro Cys Lys Pro Cys Thr Glu Cys Val Gly Leu Gln Ser Met Ser
                   85                  90                  95
   Ala Pro Cys Val Glu Ala Asp Asp Ala Val Cys Arg Cys Ala Tyr Gly
               100                 105                 110
   Tyr Tyr Gln Asp Glu Thr Thr Gly Arg Cys Glu Ala Cys Arg Val Cys
           115                 120                 125
   Glu Ala Gly Ser Gly Leu Val Phe Ser Cys Gln Asp Lys Gln Asn Thr
       130                 135                 140
   Val Cys Glu Glu Cys Pro Asp Gly Thr Tyr Ser Asp Glu Ala Asn His
   145                 150                 155                 160
   Val Asp Pro Cys Leu Pro Cys Thr Val Cys Glu Asp Thr Glu Arg Gln
                   165                 170                 175
   Leu Arg Glu Cys Thr Arg Trp Ala Asp Ala Glu Cys Glu Glu Ile Pro
               180                 185                 190
   Gly Arg Trp Ile Thr Arg Ser Thr Pro Pro Glu Gly Ser Asp Ser Thr
           195                 200                 205
   Ala Pro Ser Thr Gln Glu Pro Glu Ala Pro Pro Glu Gln Asp Leu Ile
       210                 215                 220
   Ala Ser Thr Val Ala Gly Val Val Thr Thr Val Met Gly Ser Ser Gln
   225                 230                 235                 240
   Pro Val Val Thr Arg Gly Thr Thr Asp Asn Leu Ile Pro Val Tyr Cys
                   245                 250                 255
   Ser Ile Leu Ala Ala Val Val Val Gly Leu Val Ala Tyr Ile Ala Phe
               260                 265                 270
   Lys Arg Trp Asn Ser Cys Lys Gln Asn Lys Gln Gly Ala Asn Ser Arg
           275                 280                 285
   Pro Val Asn Gln Thr Pro Pro Pro Glu Gly Glu Lys Leu His Ser Asp
       290                 295                 300
   Ser Gly Ile Ser Val Asp Ser Gln Ser Leu His Asp Gln Gln Pro His
   305                 310                 315                 320
   Thr Gln Thr Ala Ser Gly Gln Ala Leu Lys Gly Asp Gly Gly Leu Tyr
                   325                 330                 335
   Ser Ser Leu Pro Pro Ala Lys Arg Glu Glu Val Glu Lys Leu Leu Asn
                   340                 345                 350
   Gly Ser Ala Gly Asp Thr Trp Arg His Leu Ala Gly Glu Leu Gly Tyr
               355                 360                 365
   Gln Pro Glu His Ile Asp Ser Phe Thr His Glu Ala Cys Pro Val Arg
       370                 375                 380
   Ala Leu Leu Ala Ser Trp Ala Thr Gln Asp Ser Ala Thr Leu Asp Ala
   385                 390                 395                 400
   Leu Leu Ala Ala Leu Arg Arg Ile Gln Arg Ala Asp Leu Val Glu Ser
                   405                 410                 415
   Leu Cys Ser Glu Ser Thr Ala Thr Ser Pro Val
               420                 425
<210> 4
   <211> 3420
   <212> DNA
   <213> Homo sapiens
<400> 4
   agagcgagcc gagccgcggc cagctccggc gggcaggggg ggcgctggag cgcagcgcag 60
   cgcagcccca tcagtccgca aagcggaccg agctggaagt cgagcgctgc cgcgggaggc 120
   gggcgatggg ggcaggtgcc accggccgcg ccatggacgg gccgcgcctg ctgctgttgc 180
   tgcttctggg ggtgtccctt ggaggtgcca aggaggcatg ccccacaggc ctgtacacac 240
   acagcggtga gtgctgcaaa gcctgcaacc tgggcgaggg tgtggcccag ccttgtggag 300
   ccaaccagac cgtgtgtgag ccctgcctgg acagcgtgac gttctccgac gtggtgagcg 360
   cgaccgagcc gtgcaagccg tgcaccgagt gcgtggggct ccagagcatg tcggcgccgt 420
   gcgtggaggc cgacgacgcc gtgtgccgct gcgcctacgg ctactaccag gatgagacga 480
   ctgggcgctg cgaggcgtgc cgcgtgtgcg aggcgggctc gggcctcgtg ttctcctgcc 540
   aggacaagca gaacaccgtg tgcgaggagt gccccgacgg cacgtattcc gacgaggcca 600
   accacgtgga cccgtgcctg ccctgcaccg tgtgcgagga caccgagcgc cagctccgcg 660
   agtgcacacg ctgggccgac gccgagtgcg aggagatccc tggccgttgg attacacggt 720
   ccacaccccc agagggctcg gacagcacag cccccagcac ccaggagcct gaggcacctc 780
   cagaacaaga cctcatagcc agcacggtgg caggtgtggt gaccacagtg atgggcagct 840
   cccagcccgt ggtgacccga ggcaccaccg acaacctcat ccctgtctat tgctccatcc 900
   tggctgctgt ggttgtgggc cttgtggcct acatagcctt caagaggtgg aacagctgca 960
   agcagaacaa gcaaggagcc aacagccggc cagtgaacca gacgccccca ccagagggag 1020
   aaaaactcca cagcgacagt ggcatctccg tggacagcca gagcctgcat gaccagcagc 1080
   cccacacgca gacagcctcg ggccaggccc tcaagggtga cggaggcctc tacagcagcc 1140
   tgcccccagc caagcgggag gaggtggaga agcttctcaa cggctctgcg ggggacacct 1200
   ggcggcacct ggcgggcgag ctgggctacc agcccgagca catagactcc tttacccatg 1260
   aggcctgccc cgttcgcgcc ctgcttgcaa gctgggccac ccaggacagc gccacactgg 1320
   acgccctcct ggccgccctg cgccgcatcc agcgagccga cctcgtggag agtctgtgca 1380
   gtgagtccac tgccacatcc ccggtgtgag cccaaccggg gagcccccgc cccgccccac 1440
   attccgacaa ccgatgctcc agccaacccc tgtggagccc gcacccccac cctttggggg 1500
   gggcccgcct ggcagaactg agctcctctg ggcaggacct cagagtccag gccccaaaac 1560
   cacagccctg tcagtgcagc ccgtgtggcc ccttcacttc tgaccacact tcctgtccag 1620
   agagagaagt gcccctgctg cctccccaac cctgcccctg ccccgtcacc atctcaggcc 1680
   acctgccccc ttctcccaca ctgctaggtg ggccagcccc tcccaccaca gcaggtgtca 1740
   tatatggggg gccaacacca gggatggtac tagggggaag tgacaaggcc ccagagactc 1800
   agagggagga atcgaggaac cagagccatg gactctacac tgtgaacttg gggaacaagg 1860
   gtggcatccc agtggcctca accctccctc agcccctctt gccccccacc ccagcctaag 1920
   atgaagagga tcggaggctt gtcagagctg ggaggggttt tcgaagctca gcccaccccc 1980
   ctcattttgg atataggtca gtgaggccca gggagaggcc atgattcgcc caaagccaga 2040
   cagcaacggg gaggccaagt gcaggctggc accgccttct ctaaatgagg ggcctcaggt 2100
   ttgcctgagg gcgaggggag ggtggcaggt gaccttctgg gaaatggctt gaagccaagt 2160
   cagctttgcc ttccacgctg tctccagacc cccacccctt ccccactgcc tgcccacccg 2220
   tggagatggg atgcttgcct agggcctggt ccatgatgga gtcaggtttg gggttcgtgg 2280
   aaagggtgct gcttccctct gcctgtccct ctcaggcatg cctgtgtgac atcagtggca 2340
   tggctccagt ctgctgccct ccatcccgac atggacccgg agctaacact ggcccctaga 2400
   atcagcctag gggtcaggga ccaaggaccc ctcaccttgc aacacacaga cacacgcaca 2460
   cacacacaca ggaggagaaa tctcactttt ctccatgagt tttttctctt gggctgagac 2520
   tggatactgc ccggggcagc tgccagagaa gcatcggagg gaattgaggt ctgctcggcc 2580
   gtcttcactc gcccccgggt ttggcgggcc aaggactgcc gaccgaggct ggagctggcg 2640
   tctgtcttca agggcttaca cgtggaggaa tgctccccca tcctcccctt ccctgcaaac 2700
   atggggttgg ctgggcccag aaggttgtga tgaagaaaag tgggccagtg tgggaatgcg 2760
   gcaagaagga attgacttcg actgtgacct gtggggattt ctcccagctc tagacaaccc 2820
   tgcaaaggac tgttttttcc tgagcttggc cagaaggggg ccatgaggcc tcagtggact 2880
   ttccaccccc tccctggcct gttctgtttt gcctgaagtt ggagtgagtg tggctcccct     2940
   ctatttagca tgacaagccc caggcaggct gtgcgctgac aaccaccgct ccccagccca     3000
   gggttccccc agccctgtgg aagggactag gagcactgta gtaaatggca attctttgac     3060
   ctcaacctgt gatgagggga ggaaactcac ctgctggccc ctcacctggg cacctgggga     3120
   gtgggacaga gtctgggtgt atttattttc ctccccagca ggtggggagg gggtttgggg     3180
   gcttgcaagt atgttttagc atgtgtttgg ttctggggcc cctttttact ccccttgagc     3240
   tgagatggaa cccttttggc ccccgagctg ggggccatga gctccagacc cccagcaacc     3300
   ctcctatcac ctcccctcct tgcctcctgt gtaatcattt cttgggccct cctgaaactt     3360
   acacacaaaa cgttaagtga tgaacattaa atagcaaaga aagaaaaata gtacaaagag     3420